(19) Europäisches Patentamt
European Patent Office
Office européen des brevets

(11) Veröffentlichungsnummer: **0 490 219 A1**

# (12) EUROPÄISCHE PATENTANMELDUNG

(21) Anmeldenummer: **91120707.4**

(51) Int. Cl.⁵: **C07D 327/02**

(22) Anmeldetag: **02.12.91**

(30) Priorität: **13.12.90 DE 4039860**

(43) Veröffentlichungstag der Anmeldung:
**17.06.92 Patentblatt 92/25**

(84) Benannte Vertragsstaaten:
**AT BE CH DE DK ES FR GB GR IT LI LU NL SE**

(71) Anmelder: **BAYER AG**

**W-5090 Leverkusen 1 Bayerwerk(DE)**

(72) Erfinder: **Fey, Peter, Dr.**
**Am Eickhof 23**
**W-5600 Wuppertal 1(DE)**
Erfinder: **Frobel, Klaus, Dr.**
**Paul-Ehrlich-Strasse**
**W-5600 Wuppertal 1(DE)**
Erfinder: **Lenfers, Jan-Bernd, Dr.**
**Hubertusallee 9**
**W-5600 Wuppertal 1(DE)**
Erfinder: **Knorr, Andreas, Dr.**
**Trillser Graben 10**
**W-4006 Erkrath 2(DE)**
Erfinder: **Stasch, Johannes-Peter, Dr.**
**Schneewittchenweg 37**
**W-5600 Wuppertal 1(DE)**
Erfinder: **Bischoff, Erwin, Dr.**
**Pahlkestrasse 73**
**W-5600 Wuppertal 1(DE)**
Erfinder: **Dellweg, Hans-Georg, Dr.**
**In den Birken 46**
**W-5600 Wuppertal 1(DE)**
Erfinder: **Beuck, Martin, Dr.**
**Trills 7**
**W-4006 Erkrath 2(DE)**

(54) **Substituierte Dibenz-oxa-thiocinone, -12-oxide und -12,12-dioxide, Verfahren zu ihrer Herstellung und ihre Verwendung in Arzneimitteln.**

(57) Die Erfindung betrifft substituierte Dibenz-oxa-thiocinon-12-oxide und -12,12-dioxide der allgemeinen Formel I

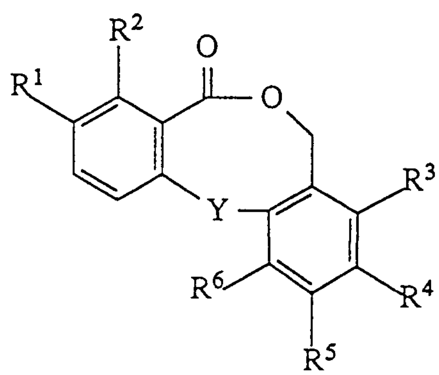

(I)

in welcher $R^1$ bis $R^6$ und Y die in der Beschreibung angegebene Bedeutung haben, Verfahren zu ihrer Herstellung und ihre Verwendung in Arzneimitteln, insbesondere in kreislaufbeeinflussenden Arzneimitteln.

EP 0 490 219 A1

Die Erfindung betrifft substituierte Dibenz-oxa-thiocinone, -12-oxide und -12,12-dioxide, Verfahren zu ihrer Herstellung und ihre Verwendung in Arzneimitteln, insbesondere in kreislaufbeeinflussenden Arzneimitteln.

Aus den Publikationen J. Org. Chem. 1981, 46, 4462-4468 und J. Am. Chem. Soc. 1981, 103, 120-127 sind bereits einige Diaryltetraoxypersulfurane bekannt.

Die vorliegende Erfindung betrifft Verbindungen der allgemeinen Formel (I)

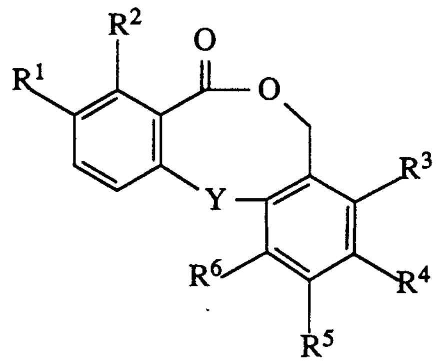

(I)

in welcher

$R^1$ und $R^6$ gleich oder verschieden sind und jeweils für Wasserstoff stehen, oder für geradkettiges oder verzweigtes Alkyl oder Alkenyl mit jeweils bis zu 10 Kohlenstoffatomen stehen, die gegebenenfalls 1- bis 3-fach durch Halogen, Azido, Imino oder durch Cycloalkyl mit 3 bis 8 Kohlenstoffatomen oder Aryl mit 6 bis 10 Kohlenstoffatomen substituiert sind, die ihrerseits gegebenenfalls bis zu 2-fach gleich oder verschieden durch Halogen, Nitro, Cyano, Hydroxy oder durch geradkettiges oder verzweigtes Alkyl, Alkoxy oder Alkoxycarbonyl mit jeweils bis zu 6 Kohlenstoffatomen substituiert sind,
oder Alkyl oder Alkenyl, die gegebenenfalls zusätzlich durch eine Gruppe der Formel $-OR^7$, $-CO-R^8$ oder $-CONR^9R^{10}$ substituiert sind,
worin

$R^7$ Wasserstoff, Cycloalkyl mit 3 bis 7 Kohlenstoffatomen oder geradkettiges oder verzweigtes Alkyl, Alkenyl oder Acyl mit jeweils bis zu 8 Kohlenstoffatomen bedeutet, die gegebenenfalls 1- bis 3-fach durch Halogen substituiertes Phenyl, Cycloalkyl mit 3 bis 7 Kohlenstoffatomen, Hydroxy, Halogen oder geradkettiges oder verzweigtes Alkoxy mit bis zu 6 Kohlenstoffatomen oder durch Carboxy, Acyl mit bis zu 6 C-Atomen oder Alkoxycarbonyl mit bis zu 6 C-Atomen substituiert sind,

$R^8$ Wasserstoff, Hydroxy, Phenoxy oder geradkettiges oder verzweigtes Alkoxy mit bis zu 8 Kohlenstoffatomen bedeutet, oder Aryl mit 6 bis 10 Kohlenstoffatomen oder Cycloalkyl mit 3 bis 8 Kohlenstoffatomen bedeutet, die gegebenenfalls durch Hydroxy, Halogen oder durch geradkettiges oder verzweigtes Alkyl, Alkoxy oder Alkoxycarbonyl mit jeweils bis zu 8 Kohlenstoffatomen substituiert sind,
geradkettiges oder verzweigtes Alkyl oder Alkenyl mit jeweils bis zu 8 Kohlenstoffatomen bedeutet, die gegebenenfalls durch Halogen Carboxy, Hydroxy oder Alkoxy, Alkoxycarbonyl oder Acyl mit jeweils bis zu 6 C-Atomen substituiert sind,

$R^9$ und $R^{10}$ gleich oder verschieden sind und Wasserstoff, geradkettiges oder verzweigtes Alkyl mit bis zu 8 Kohlenstoffatomen oder Phenyl bedeuten,
oder
$R^1$ und/oder $R^6$ direkt für eine Gruppe der Formel $-OR^7$ oder $-CO-R^8$ stehen,
worin
$R^7$ und $R^8$ die oben angegebene Bedeutung haben,

$R^2$ für Wasserstoff steht, oder
für die Gruppe $-OR^7$ steht,
worin

$R^7$ die oben angegebene Bedeutung hat, oder
für geradkettiges oder verzweigtes Alkyl oder Alkenyl mit jeweils bis zu 8 Kohlenstoffatomen steht, die gegebenenfalls durch die Gruppe $-OR^7$ substituiert sind,
worin
$R^7$ die oben angegebene Bedeutung hat, oder
für Phenyl steht, das gegebenenfalls 1- bis 3-fach durch Halogen, Nitro, Hydroxy oder durch geradkettiges oder verzweigtes Alkoxy mit bis zu 6 Kohlenstoffatomen substituiert ist,

$R^3$, $R^4$ und $R^5$ gleich oder verschieden sind und
für Wasserstoff, Nitro, Halogen, geradkettiges oder verzweigtes Alkyl mit bis zu 8 Kohlenstoffatomen stehen,

Y für ein Schwefelatom oder für die Gruppe der Formel
$>SO$ oder $>SO_2$ steht,
und deren physiologisch unbedenklichen Salze,
mit der Maßgabe, daß wenn Y für die $>SO$- oder $>SO_2$-Gruppe steht,
mindestens einer der Substituenten $R^1$ - $R^6$ verschieden von Wasserstoff sein muß.

Physiologisch unbedenkliche Salze können Salze der erfindungsgemäßen Verbindungen der allgemeinen Formel (I) mit anorganischen oder organischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Physiologisch unbedenkliche Salze können auch Salze der erfindungsgemäßen Verbindungen der allgemeinen Formel (I) (im Fall der Carbonsäuren) mit Basen sein. Als entsprechende Kationen sind dann beispielsweise physiologisch verträgliche Metall- oder Ammoniumkationen gemeint. Bevorzugt sind hierbei Alkali- bzw. Erdalkalikationen wie beispielsweise Natrium-, Kalium-, Magnesium- oder Calciumkationen, sowie Aluminium- oder Ammoniumkationen, sowie nicht-toxische substituierte Ammoniumkationen aus Aminen wie Diniedrigalkylamine, Triniedrigalkylamine, Dibenzylamin, N,N'-Dibenzylethylendiamin, N-Benzyl-$\beta$-phenylethylamin, N-Methylmorpholin oder N-Ethylmorpholin, Dihydroabietylamin, N,N'-Bis-dihydroabietylethylendiamin, N-Niedrigalkylpiperidin und andere Amine, die zur Bildung von Salzen verwendet werden können.

Die erfindungsgemäßen Verbindungen der allgemeinen Formel (I) können gegebenenfalls in stereoisomeren Formen existieren, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bevorzugt sind Verbindungen der allgemeinen Formel (I),
in welcher

$R^1$ und $R^6$ gleich oder verschieden sind und
für Wasserstoff oder
für geradkettiges oder verzweigtes Alkyl oder Alkenyl mit jeweils bis zu 8 Kohlenstoffatomen stehen, die gegebenenfalls bis zu 2-fach durch Fluor, Chlor, Brom, Jod oder durch Cyclopropyl, Cyclopentyl, Cyclohexyl oder Phenyl substituiert sind, die ihrerseits gegebenenfalls durch Fluor, Chlor, Hydroxy oder durch geradkettiges oder verzweigtes Alkyl oder Alkoxy mit jeweils bis zu 4 Kohlenstoffatomen substituiert sind, oder Alkyl oder Alkenyl, die gegebenenfalls zusätzlich durch eine Gruppe der Formel $-OR^7$, $-CO-R^8$ oder $-CONR^9R^{10}$ substituiert sind,
worin

$R^7$ Wasserstoff, Cyclopentyl, Cyclohexyl oder geradkettiges oder verzweigtes Alkyl oder Acyl mit jeweils bis zu 6 Kohlenstoffatomen bedeutet, die gegebenenfalls durch Chlor substituiertes Phenyl, Cyclopropyl, Cyclopentyl, Cyclohexyl, Hydroxy, Fluor, Chlor, Brom, geradkettiges oder verzweigtes Alkoxy mit bis zu 4 Kohlenstoffatomen oder durch Carboxy, Acyl oder Alkoxycarbonyl mit bis zu 6 C-Atomen substituiert sind,

$R^8$ Wasserstoff, Hydroxy, Phenoxy oder geradkettiges oder verzweigtes Alkoxy mit bis zu 6 Kohlenstoffatomen bedeutet, oder Phenyl, Cyclopropyl, Cyclopentyl oder Cyclohexyl bedeutet, die gegebenenfalls durch Hydroxy, Fluor, Chlor, Brom oder durch geradkettiges oder verzweigtes Alkyl oder Alkoxy mit jeweils bis zu 6 Kohlenstoffatomen substituiert sind,
geradkettiges oder verzweigtes Alkyl mit bis zu 6 Kohlenstoffatomen bedeutet, das gegebenenfalls durch Fluor, Chlor, Brom, Hydroxy, Carboxy oder durch Alkoxy, Acyl oder Alkoxycarbonyl mit bis zu 6 C-Atomen substituiert ist,

$R^9$ und $R^{10}$ gleich oder verschieden sind und Wasserstoff, geradkettiges oder verzweigtes Alkyl mit bis zu 6 Kohlenstoffatomen bedeuten,
oder
$R^1$ und/oder $R^6$ direkt für eine Gruppe der Formel $-OR^7$ oder $-CO-R^8$ stehen,
worin
$R^7$ und $R^8$ die oben angegebene Bedeutung haben,

$R^2$ für Wasserstoff oder
für die Gruppe $-OR^7$ steht,
worin
$R^7$ die oben angegebene Bedeutung hat, oder
für geradkettiges oder verzweigtes Alkyl mit bis zu 6 Kohlenstoffatomen steht, das gegebenenfalls durch die Gruppe $-OR^7$ substituiert ist,
worin
$R^7$ die oben angegebene Bedeutung hat, oder
für Phenyl steht, das gegebenenfalls durch Fluor, Chlor, Brom, Jod, Hydroxy oder geradkettiges oder verzweigtes Alkoxy mit bis zu 4 Kohlenstoffatomen substituiert ist,

$R^3$, $R^4$ und $R^5$ gleich oder verschieden sind und
für Wasserstoff, Nitro, Fluor, Chlor, Brom, Jod, geradkettiges oder verzweigtes Alkyl mit bis zu 6 Kohlenstoffatomen stehen,

Y für ein Schwefelatom oder für eine Gruppe der Formel
$>SO$ oder $>SO_2$ steht,
und deren physiologisch unbedenklichen Salze, mit der Maßgabe, daß wenn Y für
$>SO$- oder $>SO_2$-Gruppe steht,
mindestens einer der Substituenten $R^1$ - $R^6$ verschieden von Wasserstoff sein muß.

Besonders bevorzugt sind Verbindungen der allgemeinen Formel (I),
in welcher

$R^1$ und $R^6$ gleich oder verschieden sind und
für Wasserstoff, oder
für geradkettiges oder verzweigtes Alkyl oder Alkenyl mit bis zu 8 Kohlenstoffatomen stehen, das gegebenenfalls bis zu 2-fach durch Fluor, Chlor, Brom, Jod oder Phenyl substituiert ist, das seinerseits durch Chlor, Hydroxy, Methyl oder Methoxy substituiert sein kann,
Alkenyl oder Alkyl, die gegebenenfalls zusätzlich durch eine Gruppe der Formel $-OR^7$, $-CO-R^8$ oder $-CONR^9R^{10}$ substituiert sind,
worin

$R^7$ Wasserstoff, Cyclopentyl oder geradkettiges oder verzweigtes Alkyl oder Acyl mit bis zu 4 Kohlenstoffatomen bedeutet, die gegebenenfalls durch Chlor substituiertes Phenyl, Hydroxy, Chlor, Methoxy oder durch Carboxy, Acyl oder Alkoxycarbonyl mit jeweils bis zu 4 C-Atomen substituiert sind,

$R^8$ Wasserstoff, Hydroxy oder geradkettiges oder verzweigtes Alkoxy mit bis zu 4 Kohlenstoffatomen bedeutet, oder
Phenyl, Cyclopropyl, Cyclopentyl oder Cyclohexyl bedeutet, die gegebenenfalls durch Hydroxy, Fluor, Chlor, Brom oder durch geradkettiges oder verzweigtes Alkyl oder Alkoxy mit jeweils bis zu 4 Kohlenstoffatomen substituiert sind, oder
geradkettiges oder verzweigtes Alkyl mit bis zu 4 Kohlenstoffatomen bedeutet, das gegebenenfalls durch Fluor, Chlor, Brom oder durch Hydroxy, Carboxy, Alkoxy, Acyl oder Alkoxycarbonyl mit jeweils bis zu 4 C-Atomen substituiert ist,

$R^9$ und $R^{10}$ gleich oder verschieden sind und Wasserstoff oder geradkettiges oder verzweigtes Alkyl mit bis zu 4 Kohlenstoffatomen bedeuten,
oder
$R^1$ und/oder $R^6$ direkt für eine Gruppe der Formel $-OR^7$ oder $-CO-R^8$ stehen,
worin
$R^7$ und $R^8$ die oben angegebene Bedeutung haben,

$R^2$ für Wasserstoff oder
für die Gruppe $-OR^7$ steht,
worin
$R^7$ die oben angegebene Bedeutung hat,
für geradkettiges oder verzweigtes Alkyl mit bis zu 4 Kohlenstoffatomen steht, das gegebenenfalls durch die Gruppe der Formel $-OR^7$ substituiert ist,
worin
$R^7$ die oben angegebene Bedeutung hat,
für Phenyl steht, das gegebenenfalls durch Fluor, Chlor, Brom, Jod, Hydroxy oder Methoxy substituiert ist,

$R^3$, $R^4$ und $R^5$ gleich oder verschieden sind und
für Wasserstoff, Nitro, Fluor, Chlor, Brom oder Jod stehen, oder für geradkettiges oder verzweigtes Alkyl mit bis zu 4 Kohlenstoffatomen stehen,

Y für ein Schwefelatom oder für eine Gruppe der Formel
$>SO$ oder $>SO_2$ steht
und deren physiologisch unbedenklichen Salze,
mit der Maßgabe, daß wenn Y für die $>SO$- oder $>SO_2$-Gruppe steht,
mindestens einer der Substituenten $R^1$ - $R^6$ verschieden von Wasserstoff sein muß.

Außerdem wurde ein Verfahren zur Herstellung der erfindungsgemäßen Verbindungen der allgemeinen Formel (I) gefunden, dadurch gekennzeichnet, daß man
Verbindungen der allgemeinen Formel (II)

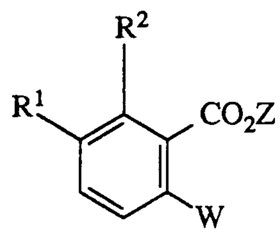

(II)

in welcher

$R^1$ und $R^2$ die oben angegebene Bedeutung haben,

W für Fluor, Chlor, Brom oder Jod, vorzugsweise für Brom steht, und

Z für Wasserstoff, $(C_1$-$C_6)$-Alkyl, Phenyl oder für ein Kalium- oder Natriumkation steht,

mit Verbindungen der allgemeinen Formel (III)

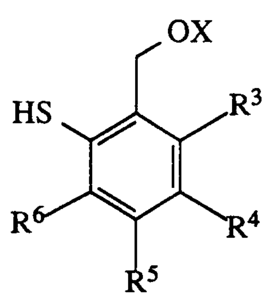

(III)

in welcher

$R^3$, $R^4$, $R^5$ und $R^6$ die oben angegebene Bedeutung haben, und

X für eine typische Hydroxyschutzgruppe, beispielsweise Tetrahydropyranyl steht,

zunächst unter Abspaltung von Halogenwasserstoffsäuren, vorzugsweise von Bromwasserstoffsäure, zu Verbindungen der allgemeinen Formel (IV)

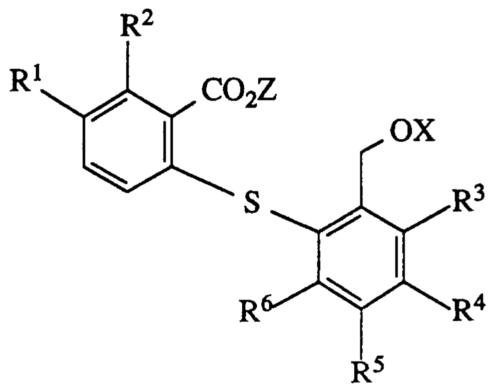

(IV)

in welcher
$R^1$, $R^2$, $R^3$, $R^4$, $R^5$, $R^6$, X und Z die oben angegebene Bedeutung haben,
in inerten Lösemitteln kondensiert, anschließend die Hydroxylgruppe nach üblicher Methode deblockiert und unter Wasserabspaltung cyclisiert, wobei sowohl die Kondensation als auch die Cyclisierung gegebenenfalls in Anwesenheit einer Base, eines Hilfsstoffes und/oder eines Katalysators durchgeführt werden kann und wobei die Substituenten $R^1$ - $R^6$ sowohl vor der Kondensation in die Verbindungen der allgemeinen Formeln (II) und (III), als auch nach der Cyclisierung in die Verbindungen der allgemeinen Formel (IV) nach üblichen Methoden, wie beispielsweise Substitution, Addition oder Eliminierung eingeführt und gegebenenfalls anschließend in andere funktionelle Gruppen überführt werden, und
im Fall, daß Y für die $>SO$- oder $>SO_2$-Gruppe steht, die
Dibenz-oxa-thiocinone (Y = S) nach üblicher Methode, beispielsweise mit Persäuren, wie m-Chlorperbenzoesäure, oder mit anderen üblichen Oxidationsmitteln wie beispielsweise Wasserstoffperoxid oder Natriummetaperjodat oxidiert werden.

Das erfindungsgemäße Verfahren wird durch folgendes Formelschema beispielhaft erläutert:

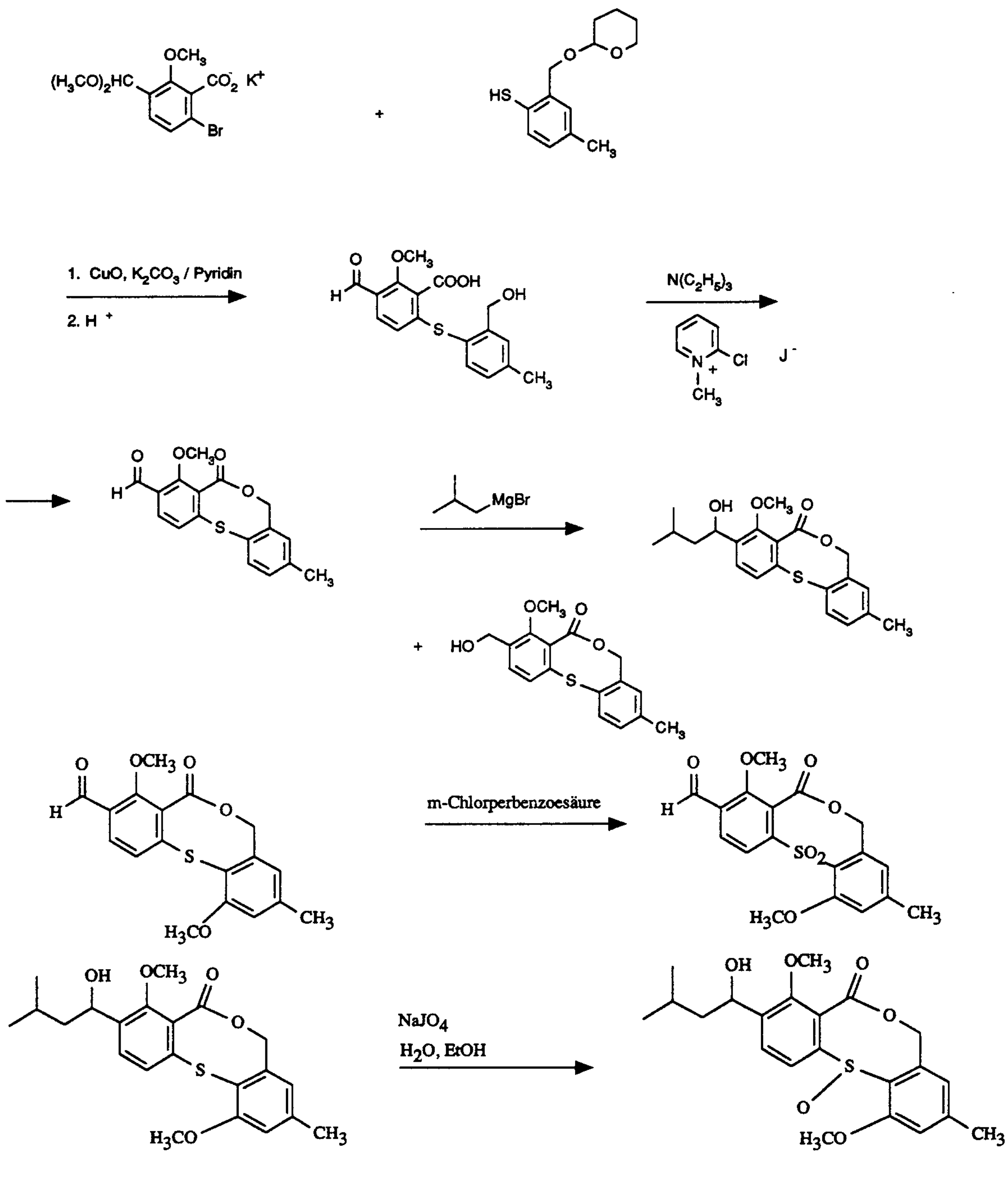


Als Lösemittel für die Kondensation, die Cyclisierung und die Oxidation können in Abhängigkeit von der jeweiligen Reaktion Wasser oder die üblichen organischen Lösemittel verwendet werden, die sich unter den Reaktionsbedingungen nicht verändern. Hierzu gehören bevorzugt Wasser, Alkohole wie Methanol, Ethanol, Propanol oder Isopropanol, oder Ether wie Diethylether, Dioxan, Tetrahydrofüran, Glykoldimethylether oder Butylmethylether, oder Ketone wie Aceton oder Butanon, oder Amide wie Dimethylformamid oder Hexamethylphosphorsäuretriamid, oder Carbonsäuren wie Essigsäure oder Propionsäure, oder Dimethylsulfoxid, Acetonitril, Essigester, oder Halogenkohlenwasserstoffe wie Methylenchlorid, Chloroform oder Tetrachlorkohlenstoff, oder Pyridin, Toluol, Xylol, Picolin oder N-Methylpiperidin. Ebenso können Gemische der genannten Lösemittel verwendet werden. Bevorzugt ist für die Kondensation Pyridin und Xylol und für die Cyclisierung Acetonitril.

Die Kondensation und die Cyclisierung werden in einem Temperaturbereich von +50 ° C bis +200 ° C, die Kondensation bevorzugt von +80 ° C bis +140 ° C, die Cyclisierung bevorzugt von +60 ° C bis + 100 ° C durchgeführt.

Die Umsetzungen können bei Normaldruck, aber auch bei erhöhtem oder erniedrigtem Druck durchgeführt werden. Im allgemeinen arbeitet man bei Normaldruck.

Bei der Durchführung der Kondensation und der Cyclisierung ist das Verhältnis der an der Reaktion beteiligten Stoffe beliebig. Im allgemeinen arbeitet man jedoch mit molaren Mengen der Reaktanden. Die Isolierung und Reinigung der erfindungsgemäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert. In einigen Fällen kann es erforderlich sein, die erfindungsgemäßen Verbindungen durch Chromatographie zu reinigen.

Als Basen eignen sich die üblichen anorganischen oder organischen Basen. Hierzu gehören bevorzugt Alkalihydroxide wie beispielsweise Natrium-, Lithium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Triethylamin, Picolin oder N-Methylpiperidin, oder 2-Chlor-N-methylpyridiniumjodid, oder Amide wie Natriumamid, Lithiumamid, Lithiumisopropylamid, oder metallorganische Verbindungen wie Butyllithium oder Phenyllithium. Bevorzugt wird für die Kondensation Kaliumcarbonat eingesetzt, während die Cyclisierung bevorzugt mit Triethylamin und 2-Chlor-N-methylpyridiniumjodid durchgeführt wird.

Als Katalysatoren werden für die Kondensation und die Cyclisierung beispielsweise Kupfersalze oder -oxide, bevorzugt Kupferoxid und Kupfer(II)acetat, oder Palladium-Katalysatoren wie z.B. $[(C_6H_5)_3P]_4Pd$, oder Alkalimetalljodide wie Kalium- oder Natriumjodid eingesetzt, die dem Reaktionsansatz in Mengen zwischen 0,5 und 150 Molprozent, bevorzugt mit 5 bei 50 Molprozent zugesetzt werden.

Als Hilfsstoffe werden bevorzugt Kondensationsmittel eingesetzt, insbesondere wenn eine Hydroxyschutzgruppe oder eine als Anhydrid aktivierte Carboxylgruppe vorliegt. Bevorzugt werden hier die üblichen Kondensationsmittel wie Carbodiimide z.B. N,N'-Diethyl-, N,N'-Dipropyl-, N,N'-Diisopropyl-, N,N'-Dicyclohexylcarbodiimid, N-(3-Dimethylaminoisopropyl)-N'-ethylcarbodiimid-Hydrochlorid oder 2-Chlor-N-methyl-pyridiniumjodid.

Die Oxidation wird in einem Temperaturbereich von 0 °C bis + 150 °C, bevorzugt von 20 °C bis + 100 °C durchgeführt.

Die Oxidation kann bei Normaldruck, erhöhtem oder erniedrigtem Druck, bevorzugt bei Normaldruck durchgeführt werden.

Die Einführung und Abspaltung der Hydroxyschutzgruppe erfolgt nach bekannten Methoden [Th. Greene, "Protective Groups in Organic Synthesis", 1. Auflage, J. Wiley & Sons, New York, 1981]. Die Schutzgruppen können beispielsweise durch saure oder basische Hydrolyse oder durch Hydrogenolyse abgespalten werden.

Die Verbindungen der allgemeinen Formeln (II) und (III) sind an sich bekannt oder können nach literaturbekannten Methoden hergestellt werden [vgl. Chem. Ber. 2555 (1928); J. Chem. Soc. Perkin Trans. I, 2973 (1983); Tietze und Eicher, Reaktionen und Synthesen im organisch chemischen Praktikum, Georg Thieme Verlag, Stuttgart, New York, 1981; W. Fuerer, H.W. Gschwend, J. Org. Chem. 44, 1133 - 1136 (1976); F.W. Vierhapper, E. Trengler, K. Kratzl, Monatshefte für Chemie, 106, 1191 - 1201 (1975); John A. Elix und Vilas Jayanthi, Aust. J. Chem. (1987), 40, 1841 - 1850].

Die Verbindungen der allgemeinen Formel (IV) sind teilweise bekannt oder neu [vgl. Collect. Czech. Chem. Commun., 39 (1), 333-54, (1974)] und können dann nach den oben aufgeführten Verfahren hergestellt werden.

Zu den oben aufgeführten allgemeinen Methoden zur Substituentenvariation gehören bevorzugt

a) die Alkylierung, d.h. die Umsetzung mit Verbindungen der allgemeinen Formel (V)

R—D (V)

in welcher

R dem Bedeutungsumfang von einem der oben aufgeführten Substituenten $R^1$ - $R^6$ entspricht, aber nicht für Wasserstoff steht, und

D eine Abgangsgruppe wie beispielsweise Chlor, Brom, Jod, $-SO_2-(C_6H_5)-p-CH_3$ bedeutet,

b) eine typische Grignard-Reaktion durch Umsetzung einer Formyl oder Acylfunktion mit Verbindungen der allgemeinen Formel (VI)

R'—MgBr (VI)

in welcher

R' für einen chemisch sinnvoll ergänzenden Rest aus dem oben aufgeführten Bedeutungsumfang

der Substituenten $R^1$-$R^6$ steht, und

c) die Halogenierung mit Verbindungen der allgemeinen Formel (VII)

E—Hal (VII)

in welcher

E für einen der oben aufgeführten Substituenten $R^1$ - $R^6$ mit der Bedeutung Fluor, Chlor, Brom, Jod oder für den Rest $-CH_2-NO_2$ steht,

d) die Oxidation, beispielsweise einer $R-CH_2-OH$-Gruppe,

worin

R die oben angegebene Bedeutung hat, mit Reagenzien wie Pyridiniumchlorochromat (PCC) oder Pyridiniumdichromat (PDC) zu den entsprechenden Oxo- oder Carboxygruppen,

wobei diese Reaktionen in einem der oben aufgeführten Lösemittel, gegebenenfalls in Anwesenheit der bereits aufgeführten Hilfsstoffe und/oder Katalysatoren, durchgeführt werden und gegebenenfalls Folgereaktionen wie Eliminierung, Reduktion, Oxidation oder Hydrolyse nach literaturbekannten Methoden angeschlossen werden können.

Die Verbindungen der Formeln (V), (VI) und (VII) sind bekannt [vgl. J. March, "Advanced Organic Chemistry", Second edition].

Die neuen Verbindungen der allgemeinen Formel (I) zeigen ein nicht vorhersehbares, wertvolles pharmakologisches Wirkspektrum. Sie beeinflussen die ANP-Freisetzung, die Kontraktionskraft des Herzens, den Tonus der glatten Muskulatur sowie den Elektrolyt- und Flüssigkeitshaushalt und wirken sowohl partiell oder vollständig digitalisantagonistisch oder digitalisagonistisch.

Sie können deshalb in Arzneimitteln zur Behandlung des pathologisch veränderten Blutdrucks, der Herzinsuffizienz, sowie als Koronartherapeutika oder als Therapeutikum bei Digitalisvergiftungen eingesetzt werden.

Darüberhinaus können sie zur Behandlung von Herzrhythmusstörungen, Niereninsuffizienz, Leberzirrhose, Aszites, Lungenödem, Hirnödem, Schwangerschaftsödem oder Glaukom eingesetzt werden.

Die antihypertensive Wirkung von 3-(1-Hydroxy-3-methyl-butyl)-4,11-dimethoxy-9-methyl-7H-dibenz[c,f]-[1,5]oxathiocin-5-on wurde an Ratten mit "reduced renal mass" Hypertonie untersucht. Die "reduced renal mass" (RRM)-Hypertonie wurde durch 5/6-Nephrektomie mit Verabreichung einer 0,5%igen Kochsalzlösung anstelle von Trinkwasser in Anlehnung an das von Hvatt et al. (1983) beschriebene Verfahren hervorgerufen.

Die erfindungsgemäßen Verbindungen senken bei dieser Hypertonieform oral den systolischen Blutdruck bei indirekter Messung an wachen Ratten.

Die erfindungsgemäßen Verbindungen stimulieren am isolierten Rattenvorhof die ANP-Freisetzung. Die ANP-Konzentration in der Badflüssigkeit wurde radioimmunologisch bestimmt [J.P. Stasch, H. Grote,S. Kazda, C. Hirth, Dynorphin stimulates the release of ANP from isolated rat arteria, Eur. J. Pharmacol. 159, 101 (1989)].

Zur vorliegenden Erfindung gehören pharmazeutische Zubereitungen, die neben nicht-toxischen, inerten pharmazeutisch geeigneten Trägerstoffen die erfindungsgemäßen Verbindungen enthalten, sowie Verfahren zur Herstellung dieser Zubereitungen.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

## Ausgangsverbindungen

### Beispiel 1

3-Methoxy-4-methyl-N-pivaloylanilin

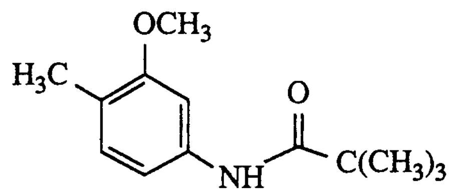

5,1 g (37 mmol) 3-Methoxy-4-methylanilin werden in 70 ml Methylenchlorid gelöst, mit 70 ml ges. $NaHCO_3$-Lösung und mit 4,6 ml (37 mmol) Pivaloylchlorid versetzt. Nach 12 Stunden unter kräftigem Rühren wird die organische Phase abgetrennt, mit 1 N Salzsäure und Wasser gewaschen, über Magnesiumsulfat getrocknet und i.V. eingeengt. Das Produkt fällt kristallin an und wird ohne weitere Reinigung weiterverarbeitet.
Ausbeute: 6,2 g (76% der Theorie) farbloser Feststoff
$C_{13}H_{19}NO_2$(221)
$^1$H-NMR (250 MHz, $CDCl_3$): $\delta$ = 1,31 (s, 9H, $C(CH_3)_3$); 2,18 (s, 3H, Ar-$CH_3$); 3,83 (s, 3H, $OCH_3$); 6,71 (dd, 1H, Ar-H); 7,03 (d, 1H, Ar-H); 7,29 (s, 1H, N-H); 7,48 (d, 1H, Ar-H).
MS (EI): 221 ($M^+$), 137

Beispiel 2

2-Methoxy-3-methyl-6-N-pivaloylaminobenzoesäure

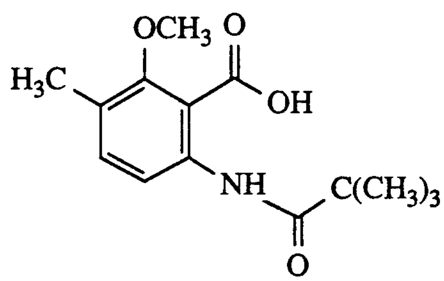

Zu einer Lösung von 59 g (0,266 mol) der Verbindung des Beispiels 1 gelöst in 800 ml abs. Tetrahydrofuran werden unter Argon 500 ml (0,8 mol) einer 1,6 M n-Butyllithium-Lsg. in Hexan bei 0°C zugetropft und 20 Stunden bei Raumtemperatur gerührt. In einem zweiten Kolben werden ebenfalls unter Argon 800 ml Tetrahydrofuran auf -70°C gekühlt und mit 140 g gepulvertem Trockeneis versetzt. Die Lösung mit der lithierten Verbindung wird jetzt langsam zu der auf -70°C kalten $CO_2$-haltigen Lösung zugetropft. Nach 10 Minuten bei -70°C wird die Lösung langsam auf 0°C aufgewärmt, mit Ether verdünnt, mit Eis hydrolysiert und mehrmals mit 1 N Natronlauge extrahiert. Die basische Lösung wird mit 2 N Salzsäure angesäuert und mit Ether extrahiert. Die organische Phase wird über Magnesiumsulfat getrocknet und eingeengt.
Ausbeute: 65,7 g (93% der Theorie), oranges Öl
$C_{14}H_9NO_4$(265)
$^1$H-NMR (250 MHz, $CDCl_3$): $\delta$ = 1,33 (s, 9H, t-Butyl); 2,32 (s, 3H, Ar-$CH_3$); 3,93 (s, 3H, $OCH_3$); 7,40 (d, 1H, Ar-H); 8,62 (d, 1H, Ar-H); 11,70 (s, 1H, -COOH).
MS (EI): 265 ($M^+$); 220, 190, 163

Beispiel 3

6-Amino-2-methoxy-3-methylbenzoesäuremethylester

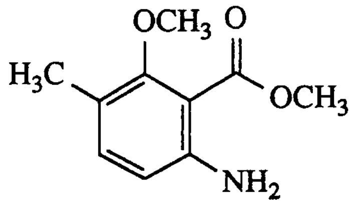

20 g (75 mmol) der Verbindung aus Beispiel 2 werden in 80 ml Methanol gelöst, mit 12 ml konz. Schwefelsäure und 9 ml (82 mmol) Orthoameisensäuremethylester versetzt und 72 Stunden unter Rückfluß erwärmt. Zur Aufarbeitung wird der Ansatz i.V. eingeengt, in Methylenchlorid aufgenommen und dreimal mit verdünnter Salzsäure extrahiert. Die wäßrige Phase wird mit konz. Natronlauge alkalisch gestellt und erschöpfend mit Methylenchlorid extrahiert. Anschließend wird die organische Phase über Magnesiumsulfat getrocknet und i.V. eingeengt. Die Titelverbindung wird ohne weitere Reinigung weiter umgesetzt.
Ausbeute: 13,1 g (89% der Theorie) braunes Öl
$C_{10}H_{13}NO_3$(195)
$^1$H-NMR (250 MHz, $CDCl_3$): $\delta$ = 2,16 (s, 3H, Ar-$CH_3$); 3,74 (s, 3H, $COOCH_3$); 3,92 (s, 3H, $OCH_3$); 4,93 (s, 2H, $NH_2$); 6,40 (d, 1H, Ar-H); 7,04 (d, 1H, Ar-H).
MS (EI): 195 ($M^+$), 163

Beispiel 4

6-Brom-2-methoxy-3-methylbenzoesäuremethylester

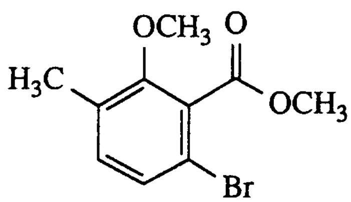

Darstellung der Diazoniumsalz-Lösung: 67,7 g (0,35 mol) der Verbindung des Beispiels 3 werden in 185 ml 48%iger Bromwasserstoffsäure und 740 ml Wasser gelöst. Unter Rühren und Kühlung (Eis-Kochsalz-Gemisch) läßt man zu dieser Lösung eine Lösung von 26 g (0.38 mol) Natriumnitrit in 150 ml Wasser so zutropfen, daß die Temperatur nicht über 5 ° C ansteigt.

Darstellung der Kupfer-(I)-bromid-Lösung: Man löst 124 g (0,5 mol) $CuSO_4$ x 5 $H_2O$ und 78 g (0,76 mol) Natriumbromid unter gelindem Erwärmen in 400 ml Wasser. Dazu tropft man langsam unter Rühren die Lösung von 65 g (0.26 mol) $Na_2SO_3$ x 7 $H_2O$ in 120 ml Wasser. Der Überstand wird nach dem Abkühlen vom farblosen Niederschlag von Kupferbromid abdekantiert. Anschließend wird der Niederschlag in 190 ml 48%iger Bromwasserstoffsäure gelöst.

Zu der auf 0 ° C gekühlten Kupfer-(I)-bromid-Lösung wird unter kräftigem Rühren die Diazoniumsalzlösung zugetropft und anschließend 12 Stunden auf 80 ° C erwärmt. Der Ansatz wird mit Methylenchlorid extrahiert, die organische Phase mit $NaHCO_3$-Lösung gewaschen, über Magnesiumsulfat getrocknet, i.V. eingeengt und säulenchromatographisch über Kieselgel mit Petrolether/Essigester 100:1 als Laufmittel gereinigt.
Ausbeute: 75,6 g (84% der Theorie), farbloses Öl
$C_{10}H_{11}BrO_3$ (259)
$^1$H-NMR (200 MHz, $CDCl_3$): $\delta$ = 2,25 (s, 3H, Ar-$CH_3$); 3,79 (s, 3H, -$COOCH_3$); 3,95 (s, 3H, $OCH_3$); 7,08 (d, 1H, Ar-H); 7,24 (d, 1H, Ar-H).
MS (EI): 260 ($M^+$), 258, 227, 229

Beispiel 5

6-Brom-3-formyl-2-methoxy-benzoesäuremethylester

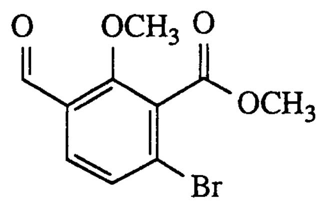

Zu 51,5g (0,2 mol) der Verbindung des Beispiels 4 gelöst in 1 l Tetrachlorkohlenstoff, werden unter Bestrahlung mit einer Quecksilberlampe 20 ml (0,4 mol) Brom gelöst in 360 ml Tetrachlorkohlenstoff bei 80°C über einen Zeitraum von 5 Stunden zugetropft. Nach weiteren 90 Minuten bei 80°C wird auf Raumtemperatur abgekühlt und mit Natriumsulfit-Lösung, Wasser und Natriumchloridlösung gewaschen. Man trocknet die organische Phase über Magnesiumsulfat und engt im Vakuum ein. Das so erhaltene Benzalbromid wird mit 100 ml konz. Schwefelsäure versetzt und 30 Minuten bei Raumtemperatur gerührt. Zur Aufarbeitung wird der Ansatz auf Eis gegossen und mit Ether extrahiert. Die Etherphase wird mit Wasser und $NaHCO_3$-Lösung gewaschen, über Magnesiumsulfat getrocknet und im Vakuum eingeengt. Die Reinigung erfolgt durch Umkristallisation aus Cyclohexan.
Ausbeute: 44,8 g (82% der Theorie) farbloser Feststoff
$C_{10}H_9BrO_4$ (273)
$^1$H-NMR (200 MHz, $CDCl_3$): $\delta$ = 4,00 (s, 3H, $OCH_3$); 4,01 (s, 3H, $OCH_3$); 7,49 (d, 1H, Ar-H); 7,78 (d, 1H, Ar-H); 10,30 (s, 1H, CHO).
MS (EI): 274 ($M^+$), 272, 243, 241, 227, 213

Beispiel 6

2-Hydroxymethyl-4-methylanilin

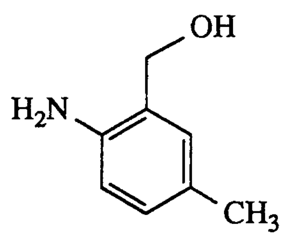

Zu einer Lösung von 100 g (0,66 mol) 2-Amino-5-methylbenzoesäure in 400 ml wasserfreiem Tetrahydrofuran tropft man unter Rückfluß 1,3 l einer 1,3 molaren Lithiumaluminiumhydridlösung in THF. Nach beendeter Zugabe wird 1,5 h weiterhin unter Rückfluß erhitzt und mit einer Lösung von 36 g Kaliumhydroxid in 145 ml Wasser bei dieser Temperatur hydrolysiert. Nach weiteren 15 min Kochen unter Rückfluß wird vom Hydroxidniederschlag heiß abgesaugt, der Niederschlag mit Essigester gewaschen, die organische Lösung im Vakuum eingeengt, in Essigester gelöst und mit verdünnter Natronlauge und Wasser gewaschen. Nach dem Trocknen mit Natriumsulfat und Einengen der Lösung wird aus Essigester umkristallisiert.
Ausbeute: 53,3 g (58% der Theorie) farbloser Feststoff
$^1$H-NMR (DMSO): $\delta$ = 2,15 (s, 3H); 4,3 (d, 2H); 4,6 (s, 2H); 4,9 (tr, 1H); 6,5 (d, 1H); 6,75 (dd, 1H); 6,85 (d, 1H).

Beispiel 7

2-Ethoxythiocarbonylthio-5-methyl-benzylalkohol

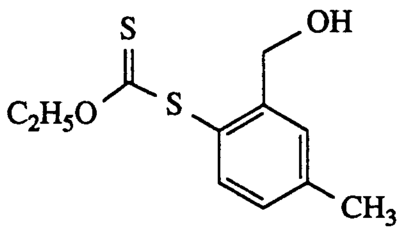

19,0 g (0,14 mol) der Verbindung aus Beispiel 6 werden in einer Mischung aus 50 ml Eis und 50 mi 32%iger Salzsäure gelöst. Unter Rühren und Kühlen (Eis-Kochsalz-Gemisch) läßt man zu dieser Lösung eine Lösung von 9,6 g (0,14 mol) Natriumnitrit in 50 ml Wasser so zutropfen, daß die Temperatur nicht über 5 ° C steigt. Die so erhaltene Diazoniumsalzlösung wird in eine auf 40 ° -50 ° C erwärmte Lösung von 33,6 g (0,2 mol) Kaliumethylxantogenat in 50 ml Wasser getropft. Nach 30 min Erwärmen auf 50 ° C läßt man abkühlen, wäscht dreimal mit Ether, wäscht die vereinigten Etherphasen mit verdünnter Natronlauge und Wasser, trocknet die Etherphase über Natriumsulfat und erhält nach Einengen im Vakuum 18,1 g Rohprodukt.
Rohausbeute: 18,1 g (53% der Theorie) Öl
$^1$H-NMR ($CDCl_3$): $\delta$ = 1,4 (tr, 3H); 2,45 (2s, 3H); 4,65 (m, 4H); 6,95 - 7,5 (m, 3H).

Beispiel 8

4-Ethoxythiocarbonylthio-3-(2-tetrahydropyranyloxymethyl)-toluol

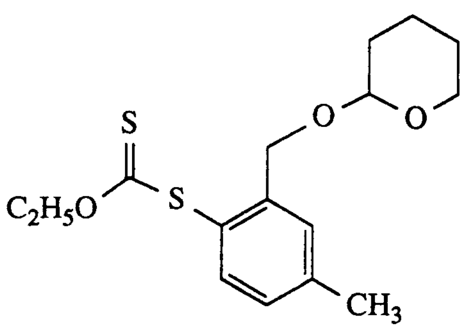

38,2 g (0,158 mol) der Verbindung des Beispiels 7, gelöst in 500ml abs. Methylenchlorid, werden mit 19,8 g (0,24 mol) Dihydropyran und einer Spatelspitze p-Toluolsulfonsäure versetzt und 14 h bei 25 ° C gerührt. Es wird mit wässriger Natriumhydrogencarbonatlösung und Wasser gewaschen, über Natriumsulfat getrocknet, im Vakuum eingeengt und an einer Kieselgelsäule mit Petrolether chromatographiert.
Ausbeute: 13,7 g (26,6% der Theorie) Öl.
$^1$H-NMR ($CDCl_3$): $\delta$ = 1,3 (tr, 3H); 1,4 - 2,0 (m, 6H); 2,4 (s, 3H); 3,55 (m, 1H); 3,95 (m, 1H); 4,5 - 5,0 (m, 5H); 7,0- 7,6 (m, 3H) ppm.

Beispiel 9

4-Methyl-2-(2-tetrahydropyranyloxymethyl)-thiophenol

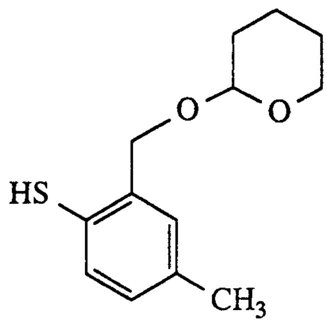

13,7 g (42 mmol) der Verbindung des Beispiels 8 werden mit 9,5 g (0,17 mol) Kaliumhydroxid in 70 ml Ethanol über Nacht unter Rückfluß erhitzt. Es wird im Vakuum eingeengt, der Rückstand in Wasser gelöst, mit Ether gewaschen, die wäßrige Phase mit Essigsäure angesäuert und mit Ether gewaschen. Diese Etherphase wird mit Wasser gewaschen, über Natriumsulfat getrocknet und im Vakuum eingeengt.
Rohausbeute: 7,0 g (70% der Theorie) Öl
$^1$H-NMR ($CDCl_3$): $\delta$ = 1,5 - 1,95 (m, 6H); 2,3 (s, 3H); 3,55 (m, 1H); 3,65 (s, 1H); 3,95 (m, 1H); 4,5 (d, 1H); 4,75 (tr, 1H); 4,8 (d, 1H); 7,0 (d, 1H); 7,15 - 7,3 (m, 2H) ppm.

Beispiel 10

2-Brom-5-dimethoxymethyl-6-methoxybenzoesäuremethylester

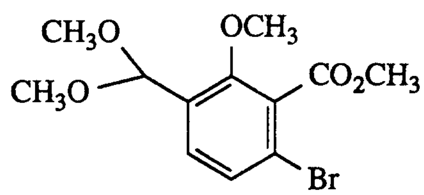

50 g (0,18 mol) der Verbindung des Beispiels 5, 20 ml (0,18 mol) Trimethylformiat und eine Spatelspitze p-Toluolsulfonsäure werden in 100 ml Methanol über Nacht unter Rückfluß erhitzt. Es wird im Vakuum eingeengt, mit Essigester aufgenommen, mit wässriger Natriumhydrogencarbonatlösung und Wasser gewaschen, über Natriumsulfat getrocknet und im Vakuum eingeengt.
Ausbeute: 50,7 g (88% der Theorie) Feststoff.
$^1$H-NMR ($CDCl_3$): $\delta$ = 3,35 (s, 6H), 3,85 (s, 3H), 3,95 (s, 3H); 5,55 (s, 1H); 7,35 (d, 1H); 7,45 (d, 1H)) ppm.

Beispiel 11

2-Brom-5-dimethoxymethyl-6-methoxybenzoesäure-Kaliumsalz

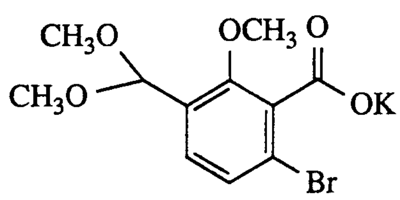

50,7 g (0,16 mol) der Verbindung aus Beispiel 10 werden mit 13,4 g (0,24 mol) Kaliumhydroxid in 200 ml Methanol und 200 ml Wasser über Nacht unter Rückfluß erhitzt. Es wird im Vakuum eingeengt und der

Rückstand in Wasser gelöst. Die Lösung wird mit Ether gewaschen und gefriergetrocknet.
Ausbeute: 50,1 g (91% der Theorie) Feststoff.

Beispiel 12

5-Formyl-6-methoxy-2-[2-hydroxymethyl-4-methyl]phenylthiobenzoesäure

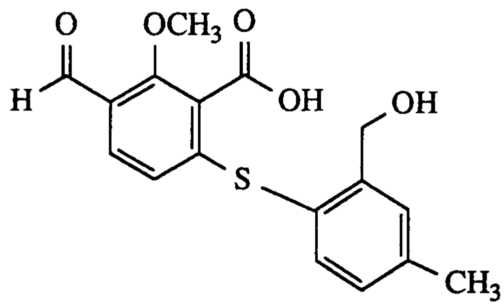

Unter Stickstoff werden zu einer Lösung von 4,97 g (1,45 mmol) der Verbindung des Beispiels 11 in 25 ml Dimethylformamid 7,0 g (29 mmol) der Verbindung des Beispiels 9, 3,2 g (23 mmol) Kaliumcarbonat und 3,7 g (46 mmol) Kupfer-II-oxid zugegeben. Die Reäktionsmischung wird in einem vorgeheizten Ölbad (120 °C) über Nacht erwärmt. Anschließend wird das Lösemittel im Vakuum abdestilliert, der Rückstand in Wasser gelöst und mit Ether gewaschen. Die wäßrige Phase wird mit verdünnter Salzsäure angesäuert, mit Essigester gewaschen, die Essigesterphase über Natriumsulfat getrocknet und im Vakuum eingeengt. Der Rückstand wird in 20 ml Methanol gelöst und mit 1 ml konz. Salzsäure über Nacht gerührt. Es wird im Vakuum eingeengt, der Rückstand in wässriger Natriumhydrogencarbonatlösung gelöst, mit Ether gewaschen, die wässrige Phase mit verdünnter Salzsäure angesäuert und mit Ether gewaschen. Diese Etherphase wird mit wässriger Natriumchloridlösung gewaschen, über Natriumsulfat getrocknet und im Vakuum eingeengt.
Ausbeute: 1,97 g (40,9% der Theorie) Schaum
$^1$H-NMR ($CDCl_3$): $\delta$ = 2,45 (s, 3H); 2,6 (br, 1H); 4,05 (s, 3H); 4,7 (s, 2H); 6,65 (d, 1H); 7,1 - 7,7 (m, 4H); 12,0 (s, 1H) ppm.

Beispiel 13

2-Dimethylthiocarbamoyloxy-3-methoxy-5-methylbenzaldehyd

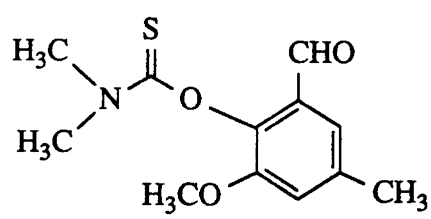

Zu 100 g (0,6 mol) 2-Hydroxy-3-methoxy-5-methylbenzaldehyd und 33,6 g (0,6 mol) Kaliumhydroxid in 400 ml Wasser werden bei 5 - 10 °C 100 ml (0,8 mol) Dimethylthiocarbamidsäurechlorid in 150 ml Tetrahydrofuran gegeben. Es wird 15 min bei 25 °C gerührt und nach Zugabe von 200 ml 10%iger wässriger Kaliumhydroxidlösung mit Essigester extrahiert, die organische Phase mit Wasser gewaschen, mit Natriumsulfat getrocknet und nach Einengen im Vakuum aus Ethanol umkristallisiert.
Ausbeute: 106,2 g (70% der Theorie)
$^1$H-NMR ($CDCl_3$): $\delta$ = 2,4 (s, 3H); 3,4 (s, 3H); 3,5 (s, 3H); 3,85 (s, 3H); 7,0 (m, 1H); 7,3 (m, 1H); 10,05 (s, 1H) ppm.

Beispiel 14

2-Dimethylcarbamoylthio-3-methoxy-5-methylbenzaldehyd

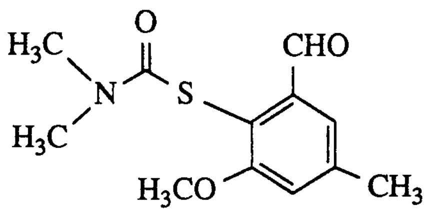

35,0 g (0,14 mol) der Verbindung aus Beispiel 13 werden in 500 ml Diphenylether 20 min bei 240 - 250 ° C gerührt. Nach dem Abkühlen auf 25 ° C wird der Diphenylether an Kieselgel mit Petrolether eluiert, anschließend wird das Produkt mit Essigester eluiert.
Ausbeute: 19,7 g (55,6% der Theorie)
Schmelzpunkt: 96-98 ° C
$^{1}$H-NMR ($CDCl_3$): $\delta$ = 2,4 (s, 3H); 3,0 (s, 3H); 3,2 (s, 3H); 3,9 (s, 3H); 7,0 (s, 1H); 7,45 (s, 1H); 10,45 (s, 1H) ppm.

Beispiel 15

2-Hydroxymethyl-6-methoxy-4-methyl-thiophenol

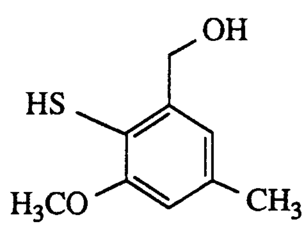

Analog Beispiel 6 werden 59 g (0,23 mol) der Verbindung aus Beispiel 14 mit 11,4 g (0,3 mol) Lithiumaluminiumhydrid reduziert.
Ausbeute: 43 g Rohprodukt (100% der Theorie)

Beispiel 16

2-Tributylstannyloxymethyl-6-methoxy-4-methyl-S-tributylstannylthiophenol

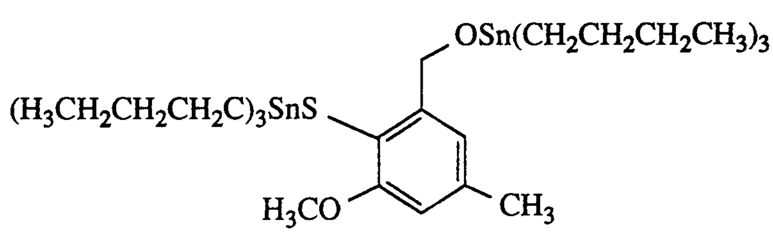

10 g (54,3 mmol) der Verbindung aus Beispiel 15 werden bei 0 bis 25 ° C mit 4 g (163 mmol)

Natriumhydrid und 31 ml (114 mmol) Tributylzinnchlorid in 300 ml Tetrahydrofuran bis zur Beendigung der Wasserstoffentwicklung gerührt und anschließend über Nacht unter Rückfluß erhitzt. Es wird vorsichtig mit Wasser hydrolysiert, mit Ether gewaschen, die vereinigten organischen Phasen mit Natriumsulfat getrocknet und im Vakuum eingeengt.
Ausbeute: 39,6 g Öl (95,6% der Theorie)
$^1$H-NMR ($CDCl_3$): $\delta$ = 0,8 - 1,7 (m, 54H); 2,35 (s, 3H); 3,2 (t, 1H); 3,85 (s, 3H); 4,75 (d, 2H); 6,6 (s, 1H); 6,8 (s, 1H) ppm.

Beispiel 17

5-Formyl-6-methoxy-2-[2-hydroxymethyl-6-methoxy-methylphenylthio]benzoesäuremethylester

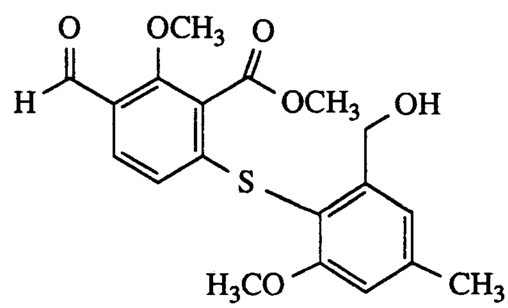

1,08 g (3,94 mmol) der Verbindung aus Beispiel 5 und 2 g (2,6 mmol) der Verbindung aus Beispiel 16 und 30 mg Tetrakistriphenylphosphinpalladium werden unter Stickstoffatmosphäre in 40 ml Toluol 50 h unter Rückfluß erhitz. Nach dem Einengen im Vakuum wird in Acetonitril aufgenommen und fünfmal mit Pentan gewaschen. Die Acetonitrilphase wird im Vakuum eingeengt und das verbleibende Öl (1,33 g ) an einer Kieselgelsäule mit Essigester/Petrolether 1:5 chromatographiert.
Ausbeute: 360 mg Feststoff (36,2% der Theorie)
$^1$H-NMR ($CDCl_3$): $\delta$ = 2,45 (s, 3H); 3,8 (s, 3H); 4,0 (2s, 6H); 4,75 (d, 2H); 6,55 (d, 1H); 6,75 (s, 1H); 7,05 (s, 1H); 7,6 (d, 1H); 10,2 (s, 1H) ppm.

Als Nebenprodukt werden 212 mg (26,1% der Theorie) 5-Formyl-6-methoxy-2-[2-formyl-6-methoxy-4-methylphenylthio]benzoesäuremethylester

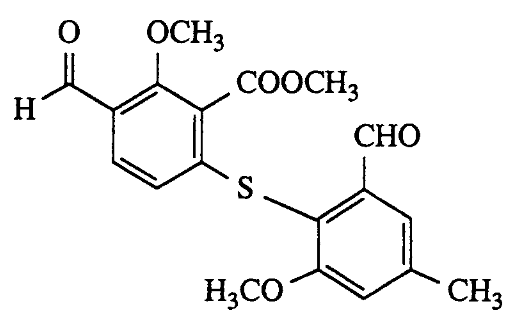

erhalten.

$^1$H-NMR ($CDCl_3$): $\delta$ = 2,5 (s, 3H); 3,8 (s, 3H); 4,0 (2s, 6H); 6,55 (d, 1H); 7,05 (s, 1H); 7,5 (s, 1H); 7,65 (d, 1H); 10,25 (s, 1H); 10,6 (s, 1H) ppm.

Beispiel 18

5-Formyl-6-methoxy-2-[2-hydroxymethyl-6-methoxy-methylphenylthio]benzoesäure

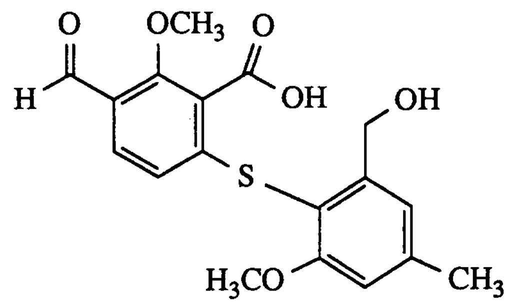

302 mg (0,8 mol) der Verbindung aus Beispiel 17 werden in 4,6 ml Methanol gelöst und 1 h mit 36 mg p-Toluolsulfonsäure bei 25°C gerührt. Nach Zugabe von 0,45 g Kaliumhydroxid wird über Nacht unter Rückfluß erhitzt. Es wird im Vakuum eingeengt, mit verdünnter Salzsäure auf pH = 3 eingestellt und mit Essigester gewaschen. Die organische Phase wird mit Natriumsulfat getrocknet und im Vakuum eingeengt.
Ausbeute: 273 mg Feststoff (94% der Theorie)
$^1$H-NMR (DMSO): $\delta$ = 2,4 (s, 3H); 3,7 (s, 3H); 3,95 (s, 3H); 4,5 (s, 2H); 6,35 (d, 1H); 6,95 (s, 1H); 7,1 (s, 1H); 7,55 (d, 1H); 10,1 (s, 1H) ppm.

Herstellungsbeispiele

Beispiel I

3-Formyl-4-methoxy-9-methyl-7H-dibenz[c,f][1,5]oxathiocin-5-on

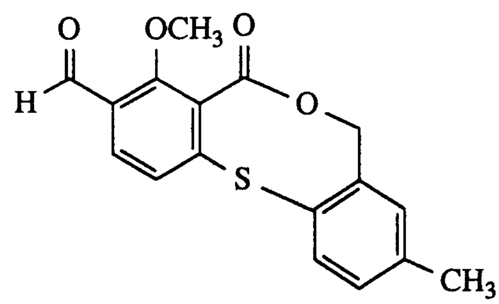

Zu einer Lösung von 5,3 g (23,7 mmol) 2-Chlor-1-methyl-pyridiniumiodid in 380 ml absolutem Acetonitril wird bei 80°C eine Lösung von 1,97 mg (5,92 mmol) der Verbindung des Beispiels 12 und 6,5 ml (47,3 mmol) Triethylamin in 380 ml absolutem Acetonitril über einen Zeitraum von 6 h zugetropft. Nach einer weiteren Stunde bei 80°C wird im Vakuum eingeengt, der Rückstand in Dichlormethan aufgenommen, mit Wasser gewaschen, über Magnesiumsulfat getrocknet und eingeengt. Die Reinigung erfolgt säulenchromatographisch an Kieselgel mit Petrolether/Essigester 2:1.
Ausbeute: 700 mg (37,6% der Theorie) Feststoff
$^1$H-NMR ($CDCl_3$): $\delta$ = 2,3 (s, 3H); 4,1 (s, 3H); 5,25 (s, 2H); 6,95 (s, 1H); 7,1 (d, 1H); 7,4 (d, 1H); 7,55 (d, 1H); 7,9 (d, 1H) ppm.

Beispiel II

3-(1-Hydroxy-3-methylbutyl)-4-methoxy-9-methyl-7H-dibenz[c,f][1,5]oxathiocin-5-on

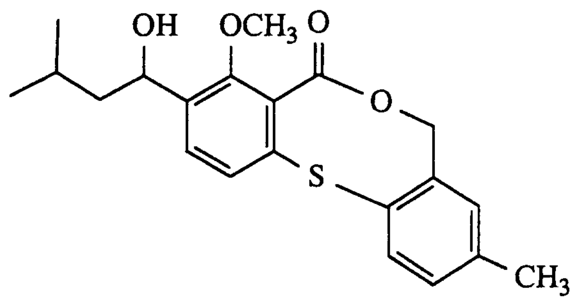

660 mg (2,1 mmol) der Verbindung des Beispiels I in 10 ml absolutem Tetrahydrofuran werden unter Argon bei 0 ° C mit 1,2 ml(2,3 mmol) einer 2 M Isobutyryl-magnesiumbromid-Lösung in Tetrahydrofuran versetzt und 1 h bei 25 ° C gerührt. Nach Zugabe von 2 ml 1 N Salzsäure wird mit Methylenchlorid verdünnt, mit Wasser gewaschen, getrocknet und eingedampft. Nach chromatographischer Reinigung an Kieselgel Si60 (Petrolether/Essigester 5:1) fallen 121 mg (15% der Theorie) des Hauptprodukts als farbloser Feststoff an.

$^1$H-NMR ($CDCl_3$): $\delta$ = 1,0 (t, 6H); 1,45 (m, 1H); 1,6-1,9 (m, 2H); 1,95 (d, 1H); 2,3 (s, 3H); 3,95 (s, 1H); 5,1 (m, 1H); 5,15 (s, 2H); 6,85 (s, 1H); 7,1 (d, 1H); 7,35 (d, 1H); 7,45 (d, 1H) 7,6 (d, 1H);

Als Nebenprodukt werden 322 mg 3-Hydroxymethyl-4-methoxy-9-methyl-7H-dibenz-[c,f][1,5]oxathiocin-5-on (Beispiel III)isoliert.

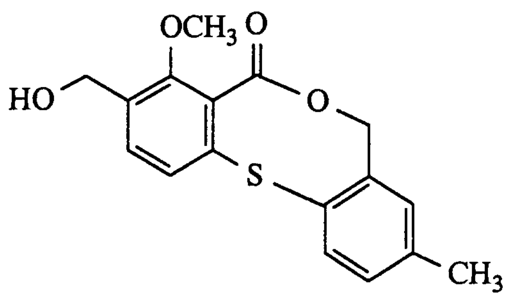

$^1$H-NMR ($CDCl_3$): $\delta$ = 2,0 (t, 1H); 2,3 (s, 3H); 3,95 (s, 3H); 4,8 (d, 2H); 5,15 (s, 2H); 6,85 (s, 1H); 7,1 (d, 1H); 7,35 - 7,6 (m, 3H) ppm.

Beispiel IV

3-Formyl-4,11-dimethoxy-9-methyl-7H-dibenz[c,f][1,5]oxathiocin-5-on

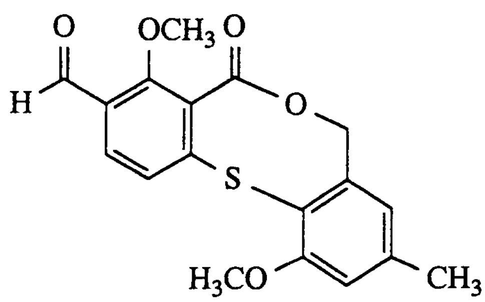

Analog Beispiel I werden aus 128 mg (0,35 mmol) der Verbindung aus Beispiel 18 9,9 mg Produkt erhalten.

Ausbeute: 8% der Theorie
$^1$H-NMR ($CDCl_3$): δ = 2,3 (s, 3H); 3,9 (s, 3H); 4,1 (s, 3H); 5,2 (s, 2H); 6,55 (s, 1H); 6,75 (s, 1H); 7,55 (d, 1H); 7,95 (d, 1H); 10,4 (s, 1H) ppm.

Beispiel V

3-(1-Hydroxy-3-methylbutyl)-4,11-dimethoxy-9-methyl-7H-dibenz[c,f][1,5]oxathiocin-5-on

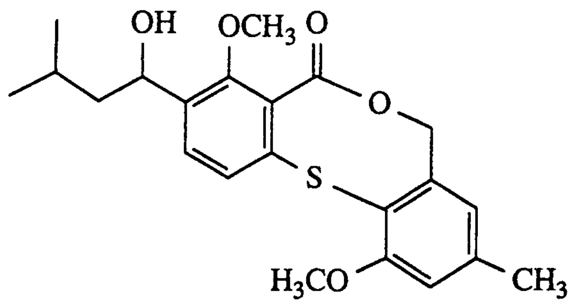

Analog Beispiel II erhält man aus 9,9 mg (0,03 mmol) der Verbindung aus Beispiel IV 4 mg farblosen Feststoff.
Ausbeute: 34% der Theorie
$^1$H-NMR ($CDCl_3$): δ = 0,9 (m, 6H), 1,5 (m, 3H); 2,3 (s, 3H); 3,9 (s, 3H); 4,0 (s, 3H); 4,95 (m, 1H); 5,1 (m, 2H); 6,5 (s, 1H); 6,8 (s, 1H); 7,45 (d, 1H); 7,6 (d, 1H) ppm.
Als Nebenprodukt werden 3,5 mg (30% der Theorie) 3-Hydroxymethyl-4,11-dimethoxy-9-methyl-7H-dibenz-[c,f][1,5]oxathiocin-5-on (Beispiel VI) isoliert.

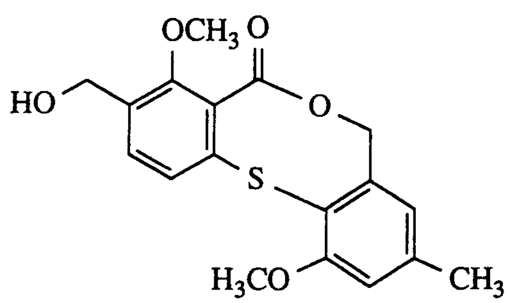

$^1$H-NMR ($CDCl_3$) :δ = 2,35 (s, 3H); 3,9 (s, 3H); 4,0 (s, 3H); 4,75 (s, 2H); 5,1 (s, 2H); 6,5 (s, 1H); 6,85 (s, 1H); 7,5 (q, 2H) ppm.

Beispiel VII

3-(1-Hydroxy-2-methylpropyl)-4-methoxy-9-methyl-7H-dibenz[c,f][1,5]oxathiocin-5-on

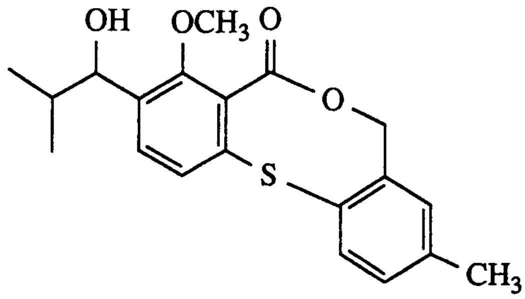

Analog Beispiel II erhält man aus 250 mg (0,8 mmol) der Verbindung aus Beispiel I und 0,44 ml (0,88 mmol) einer 2-molaren Lösung von Isopropylmagnesiumchlorid in Ether 63,8 mg der Titelverbindung.
Ausbeute: 22,4% der Theorie
$^1$H-NMR ($CDCl_3$): $\delta$ = 0,8 (d, 3H); 1,0 (d, 3H); 2,0 (m, 1H); 2,3 (s, 3H); 3,95 (s, 3H); 4,7 (m, 1H); 5,15 (s, 2H); 6,9 (s, 1H); 7,1 (d, 2H); 7,4 (m, 2H); 7,55 (d, 2H) ppm.

Beispiel VIII

3-Carboxy-4,11-dimethoxy-9-methyl-7H-dibenz[c,f][1,5]oxathiocin-5-on

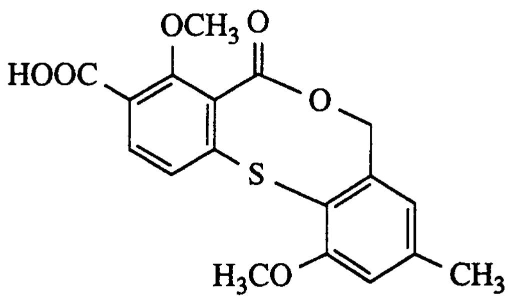

560 mg (1,62 mmol) der Verbindung aus Beispiel VI werden bei 25°C in 20 ml Dimethylformamid mit 9,13 g (24,3 mmol) Pyridiniumdichromat über Nacht gerührt. Es wird auf Wasser gegossen, mit Ether gewaschen, die Etherphase mit Wasser gewaschen, über Natriumsulfat getrocknet, und nach dem Einengen im Vakuum an Kieselgel mit Methylenchlorid/Methanol 10:1 chromatographiert.
Ausbeute: 310 mg Feststoff (53% der Theorie)
$^1$H-NMR (DMSO): $\delta$ = 2,25 (s, 3H); 3,85 (2d, 6H); 5,1 (s, 2H); 6,65 (s, 1H); 6,95 (s, 1H); 7,3 (d, 1H); 7,55 (d, 1H) ppm.

Beispiel IX

3-Formyl-4,11-dimethoxy-9-methyl-7H-dibenz[c,f][1,5]-oxathiocin-5-on-12,12-dioxid

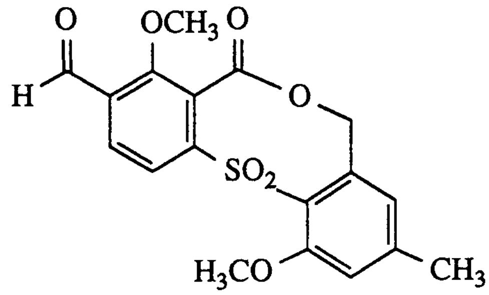

50 mg (0,15 mmol) der Verbindung aus Beispiel IV werden in 10 ml Methylenchlorid mit 65 mg (0,32 mmol) Metachlorperbenzoesäure (80%ig) bei 25 ° C gerührt. Es wird mit Methylenchlorid verdünnt und die organische Phase mehrfach mit Natriumthiosulfatlösung, Natriumcarbonatlösung und Wasser gewaschen. Nach dem Trocknen mit Natriumsulfat und Einengen der Methylenchloridlösung erhält man 56 mg farblosen Schaum.
Ausbeute: 97,6% der Theorie
$^{1}$H-NMR ($CDCl_3$): $\delta$ = 2,4 (s, 3H); 3,95 (s, 3H); 4,15 (s, 3H); 5,45 (m, 2H); 6,8 (s, 1H); 6,9 (s, 1H); 8,1 (d, 1H); 8,2 (d, 1H) ppm.

Beispiel X

3-(1-Hydroxy-3-methylbutyl)-4,11-dimethoxy-9-methyl-7H-dibenz[c,f][1,5]-oxathiocin-5-on-12,12-dioxid

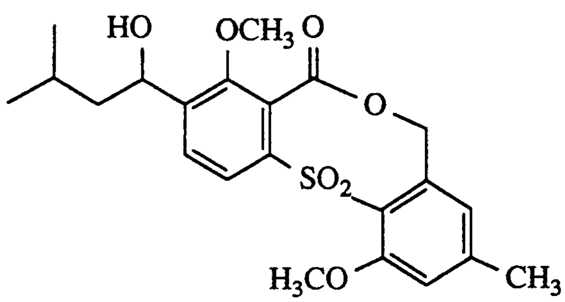

Analog Beispiel II erhält man aus 50 mg der Verbindung aus Beispiel IX 8 mg der Titelverbindung.
Ausbeute: 13,8% der Theorie
$^{1}$H-NMR ($CDCl_3$): $\delta$ = 1,0 (m, 6H); 1,4 - 2,0 (m, 3H); 2,4 (s, 3H); 3,95 (s, 3H); 4,0 (s, 3H); 5,1 (m, 1H); 5,3 (m, 2H); 6,7 (s, 1H); 6,9 (s, 1H); 7,8 (d, 1H); 8,05 (d, 1H) ppm.
Weiterhin werden 18,1 mg

Beispiel XI

3-Hydroxymethyl-4,11-dimethoxy-9-methyl-7H-dibenz[c,f]-[1,5]oxathiocin-5-on-12,12-dioxid

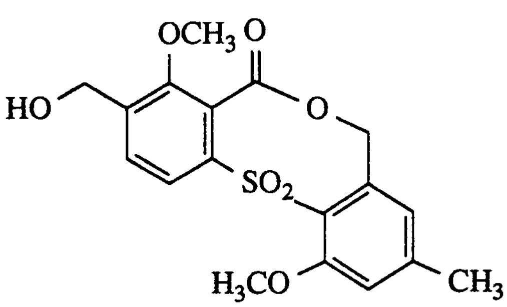

isoliert.
Ausbeute: 35,9% der Theorie
$^1$H-NMR ($CDCl_3$): δ = 2,35 (s, 3H); 3,95 (s, 3H); 4,0 (s, 3H); 4,85 (m, 2H); 5,3 (m, 2H); 6,7 (s, 1H); 6,85 (s, 1H); 7,8 (d; 1H); 8,1 (d, 1H) ppm.

Beispiel XII

3-(1-Hydroxy-2-methylpropyl)-4-methoxy-9-methyl-7H-dibenz[c,f][1,5]oxathiocin-5-on-12,12-dioxid

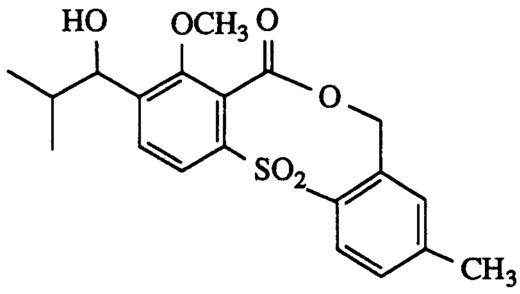

Analog Beispiel IX werden aus 61,4 mg (0,17 mmol) der Verbindung aus Beispiel VII 52 mg der Titelverbindung erhalten.
Ausbeute: 77% der Theorie
$^1$H-NMR ($CDCl_3$): δ = 0,9 (2d, 6H); 2,0 (m, 1H); 2,4 (s, 3H); 4,0 (s, 3H); 4,8 (m, 1H); 5,4 (m, 2H); 7,2 (s, 1H); 7,35 (d, 1H); 7,75 (d, 1H); 8,05 (d, 1H); 8,15 (d, 1H) ppm.
MS (CI): 408 ($M+NH_4^+$); 390; 373; 364; 347
Die Verbindung aus Beispiel II wird unter HPLC-Bedingungen (24 bar) an einer CHIRACEL$^R$ OJ-Säule mit Hexan/2-Propanol-Gemischen in die beiden Enantiomeren (Beispiel XIII und Beispiel XIV) getrennt:

Beispiel XIII

(+)-3-(1-Hydroxy-3-methylbutyl)-4-methoxy-9-methyl-7H-dibenz[c,f][1,5]oxathiocin-5-on

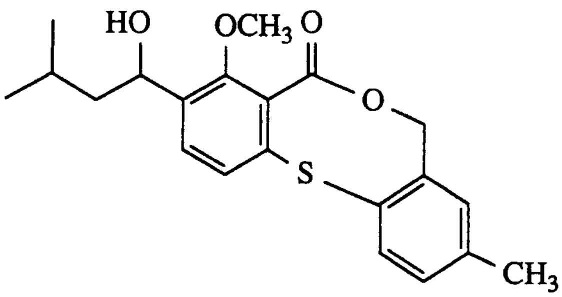

$[\alpha]^{20}/_D$ = + 22,2° (c=1 in 2-Propanol)

Beispiel XIV

(-)-3-(1-Hydroxy-3-methylbutyl)-4-methoxy-9-methyl-7H-dibenz[c,f][1,5]oxathiocin-5-on

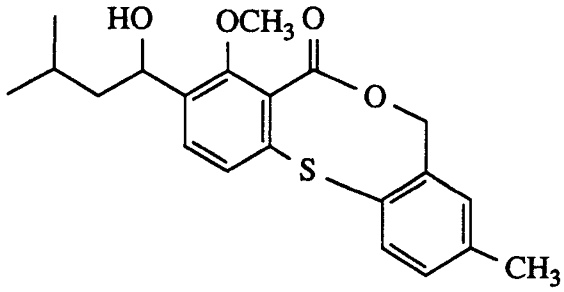

$[\alpha]^{20}/_D$ = - 20,1° (c = 1 in 2-Propanol)

Beispiel XV

8-Brom-3-(1-hydroxy-3-methylbutyl)-4,11-dimethoxy-9-methyl-7H-dibenz[c,f][1,5]oxathiocin-5-on

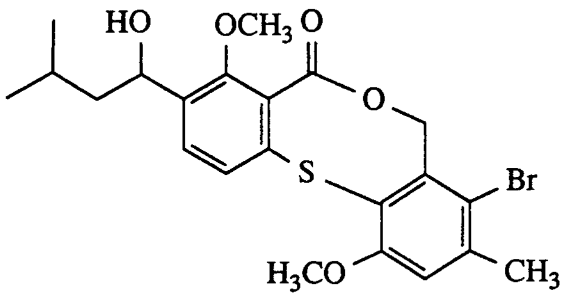

Zu einer Lösung von 50 mg (0,13 mmol) der Verbindung aus Beispiel V und 33,6 mg (0,13 mmol) Eisen-(III)-chloridtrihydrat in 1 ml Ethanol/Wasser (3:1) gibt man 12,8 µl (0,25 mmol) Brom und rührt bei 25°C über Nacht. Nach Verdünnen mit Methylenchlorid und Waschen mit 10%iger Kaliumjodidlösung und Wasser wird mit Natriumsulfat getrocknet, eingeengt und an Kieselgel mit Essigester/Petrolether (1:5) chromatographiert.
Ausbeute: 37 mg Schaum (62% der Theorie)
$^1$H-NMR ($CDCl_3$): $\delta$ = 1,0 (m, 6H); 1,4 - 1,9 (m, 3H); 2,0 (d, 1H); 2,4 (s, 3H); 3,9 (s, 3H); 4,0 (s, 3H); 5,1 (m, 1H); 5,6 (s, 2H); 6,8 (s, 1H); 7,45 (d, 1H); 7,6 (d, 1H)ppm.

Beispiel XVI

3-(1-Hydroxy-3-methylbutyl)-4,11-dimethoxy-9-methyl-7H-dibenz[c,f][1,5]oxathiocin-5-on-12-oxid

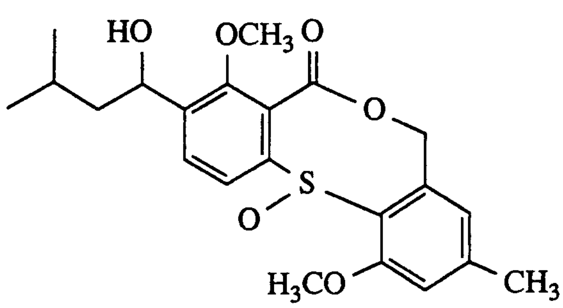

Analog Beispiel IX werden 32 mg (0.08 mmol) der Verbindung aus Beispiel V mit 16 mg (0.08 mmol)

Metachlorperbenzoesäure (80 bis 90 %ig) umgesetzt.

Neben 7,7 mg Edukt (Beispiel I) und 10 mg des Sulfons (Beispiel X) werden 22 mg der Titelverbindung erhalten.

| | |
|---|---|
| Ausbeute: | 65,7 % der Theorie |
| $^1$H-NMR ($CDCl_3$): $\delta$ = | 0,95 (m, 6H); 1,4-1,9 (m, 3H); 2,3 (s, 3H); 3,75 (s, 3H); 3,95 (s, 3H); 5,1 (m, 1H); 5,45 (q, 2H); 6,65 (s, 1H); 6,7 (s, 1H); 7,7-8,0 (m, 2H) ppm. |
| MS(EI): | 418; 402; 357 |

Analog zu Beispiel XIII und XIV wurde die Verbindung aus Beispiel XV in die beiden Enantiomeren (Beispiele XVII und XVIII) getrennt:

Beispiel XVII

(+)-8-Brom-3-(1-hydroxy-3-methylbutyl)-4,11-dimethoxy-9-methyl-7H-dibenz[c,f][1,5]oxathiocin-5-on

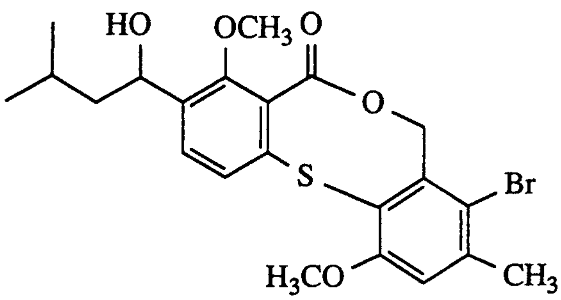

$[\alpha]^{20}/_D$ = +8,76° (c=1 in Methanol)

Beispiel XVIII

(-)-8-Brom-3-(1-hydroxy-3-methylbutyl)-4,11-dimethoxy-9-methyl-7H-dibenz[c,f][1,5]oxathiocin-5-on

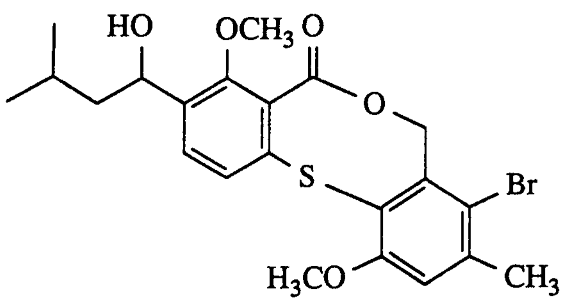

$[\alpha]^{20}/_D$ = -4,87° (c = 1 in Methanol)

Beispiel XIX

(+)-8-Brom-3-(1-hydroxy-3-methylbutyl)-4,11-dimethoxy-9-methyl-7H-dibenz[c,f][1,5]oxathiocin-5-on-12,12-dioxid

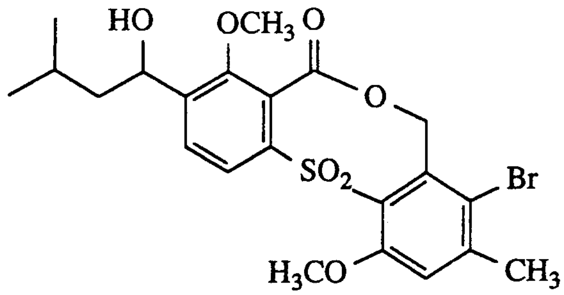

Analog Beispiel IX werden 403,8 mg (0,84 mmol) der Verbindung aus Beispiel XVII mit 170,5 mg (0,84 mmol) Metachlorperbenzoesäure (80-90 %ig) umgesetzt und an 75 g Kieselgel mit Dichlormethan/Essigester (20:1) chromatographiert.

Neben 43,7 mg Edukt (10 % der Theorie) werden 35 mg (8 % der Theorie) der Titelverbindung und 280 mg (67 % der Theorie) der Verbindung aus Beispiel XX erhalten.

MS(EI): 514, 512 ($M^+$) 457, 455

$[\alpha]^{20}/_D$ = +12,63° (c = 1 in Methanol)

Beispiel XX

(+)-8-Brom-3-(1-hydroxy-3-methylbutyl)-4,11-dimethoxy-9-methyl-7H-dibenz[c,f][1,5]oxathiocin-5-on-12-oxid (Diastereomerengemisch)

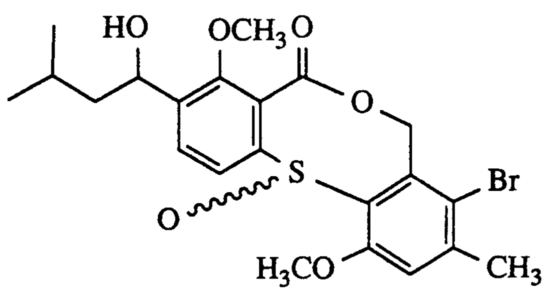

Die Titelverbindung wurde wie unter Beispiel XIX beschrieben erhalten.

Beispiel XXI

(-)-8-Brom-3-(1-hydroxy-3-methylbutyl)-4,11-dimethoxy-9-methyl-7H-dibenz[c,f][1,5]oxathiocin-5-on-12,12-dioxid

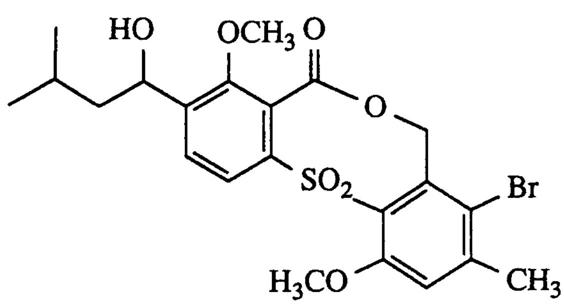

Analog Beispiel XIX werden aus 445 mg (0,952 mmol) der Verbindung aus Beispiel XVIII neben 7 % Edukt 113 mg (23,8 % der Theorie) der Titelverbindung erhalten.
$[\alpha]^{20}/_D$ = -18,03° (c = 1 in Methanol)
Weiterhin wurden 330 mg (69,5 % der Theorie) der Verbindung aus

Beispiel XXII

(-)-8-Brom-3-(1-hydroxy-3-methylbutyl)-4,11-dimethoxy-9-methyl-7H-dibenz[c,f][1,5]oxathiocin-5-on-12-oxid (Diastereomerengemisch)

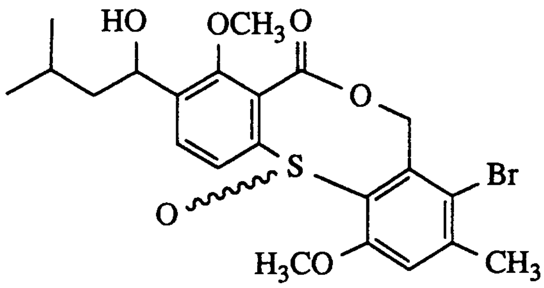

erhalten.

Beispiele XXIII - XXXI:

Analog zu Beispiel XIX werden aus 1,39 g (3,74 mmol) der Verbindung aus Beispiel II nach Chromatographie an 150 g Kieselgel mit Essigester/Petrolether 1:4 neben 242,1 mg (17,3 % der Theorie) des Edukts und einer Mischfraktion (657 mg) 291,8 mg (20,1 % der Theorie) des racemischen Sulfoxids aus Beispiel XXIII erhalten:

Beispiel XXIII

(±)-3-(1-Hydroxy-3-methylbutyl)-4-methoxy-9-methyl-7H-dibenz[c,f][1,5]oxathiocin-5-on-12-oxid (1. Diastereomer)

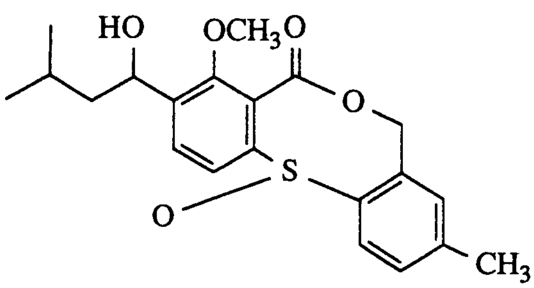

MS(EI): 388 ($M^+$) 331,151
Schmelzpunkt: 146-160° C
Durch Chromatographie der Mischfraktion an 150 g Kieselgel mit Dichlormethan/Essigester 20:1 werden 290 mg (20 % der Theorie) des racemischen Sulfons aus Beispiel XXIV und 304 mg (20,9 % der Theorie) des racemischen Sulfoxids aus Beispiel XXV (2. Diastereomer) erhalten:

Beispiel XXIV

(±)-3-(1-Hydroxy-3-methylbutyl)-4-methoxy-9-methyl-7H-dibenz[c,f][1,5]-oxathiocin-5-on-12,12-dioxid

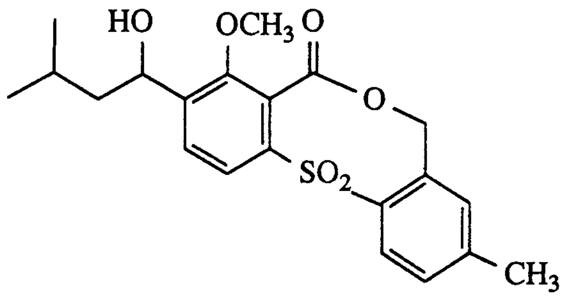

MS(EI) 422 ($M+NH_4^+$), 404 ($M^+$), 378, 364, 347

Beispiel XXV

(±)-3-(1-Hydroxy-3-methylbutyl)-4-methoxy-9-methyl-7H-dibenz[c,f][1,5]-oxathiocin-5-on-12-oxid (2. Diastereomer)

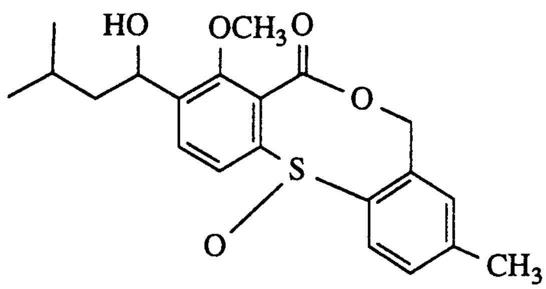

MS(DCI) 389 ($M+H^+$), 331
Schmelzpunkt: 166°-170°C
Die racemischen Verbindungen der Beispiele XXIII bis XXV wurden analog zu Beispiel XIII/XIV in die Enantiomeren getrennt:

Beispiel XXVI

(+)-3-(1-Hydroxy-3-methylbutyl)-4-methoxy-9-methyl-7H-dibenz[c,f][1,5]oxathiocin-5-on-12-oxid (1. Diastereomer).

$[\alpha]^{20}/_D$ = +260,54° (c = 1 in Methanol)

Beispiel XXVII

(-)-3-(1-Hydroxy-3-methylbutyl)-4-methoxy-9-methyl-7H-dibenz[c,f][1,5]oxathiocin-5-on-12-oxid (1. Diastereomer).
$[\alpha]^{20}/_D$ = -256,66° (c = 1 in Methanol)

Beispiel XXVIII

(+)-3-(1-Hydroxy-3-methylbutyl)-4-methoxy-9-methyl-7H-dibenz[c,f][1,5]oxathiocin-5-on-12-oxid (2. Diastereomer).
$[\alpha]^{20}/_D$ = +197,09° (c = 1 in Methanol)

Beispiel XXIX

(-)-3-(1-Hydroxy-3-methylbutyl)-4-methoxy-9-methyl-7H-dibenz[c,f[1,5]oxathiocin-5-on-12-oxid (2. Diastereomer).

$[\alpha]^{20}/_D$ = -202,02° (c = 1 in Methanol)

Beispiel XXX

(+)-3-(1-Hydroxy-3-methylbutyl)-4-methoxy-9-methyl-7H-dibenz[c,f][1,5]oxathiocin-5-on-12,12-dioxid. $[\alpha]^{20}/_D$ = +1,47° (c = 1 in Methanol)

Beispiel XXXI

(-)-3-(1-Hydroxy-3-methylbutyl)-4-methoxy-9-methyl-7H-dibenz[c,f][1,5]oxathiocin-5-on-12,12-dioxid. $[\alpha]^{20}/_D$ = -1,52° (c = 1 in Methanol)

Beispiel XXXII

8-Brom-4,11-dimethoxy-9-methyl-3-(3-methylbutan-1-oyl)-7H-dibenz[c,f][1,5]-oxathiocin-5-on

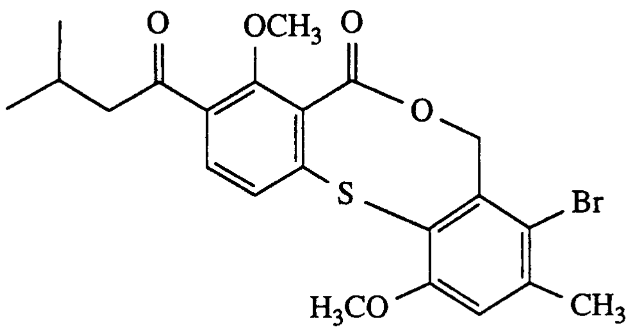

Zu einer Losung von 35 mg (0,07 mmol) der Verbindung aus Beispiel XV in 4 ml Dichlormethan gibt man 31 mg (0,15 mmol) Pyridiniumchlorochromat und rührt 2 Stunden bei Raumtemperatur. Die Reaktionslösung wird auf Kieselgel aufgetragen, mit Dichlormethan eluiert und an Kieselgel mit Petrolether/Essigester 1:3 chromatographiert.

Ausbeute: 31,1 mg (93 % der Theorie)
MS(EI): 480, 478 ($M^+$), 452, 450, 425, 422

Beispiel XXXIII

(±)-3-(1-Hydroxy-3-methyl-3-buten-1-yl)-4,11-dimethoxy-9-methyl-7H-dibenz[c,f][1,5]oxathiocin-5-on

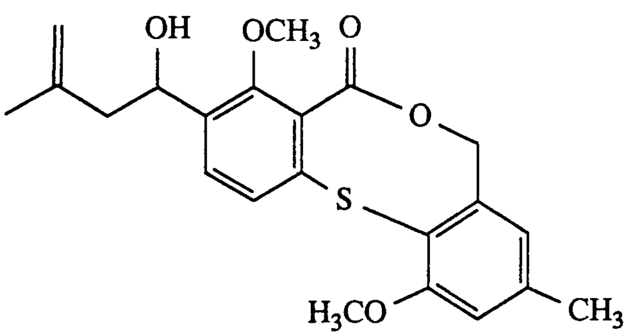

Analog Beispiel II erhält man aus 70 mg (0,2 mmol) der Verbindung des Beispiels IV und Methallylmagnesiumchlorid 83 mg der Titelverbindung.
Ausbeute: 100 % der Theorie

MS(EI): 400 ($M^+$), 345, 327, 315, 299

Beispiel XXXIV

(±)-3-(1-Hydroxy-3-methyl-3-buten-1-yl)-4-methoxy-9-methyl-7H-dibenz[c,f][1,5]oxathiocin-5-on

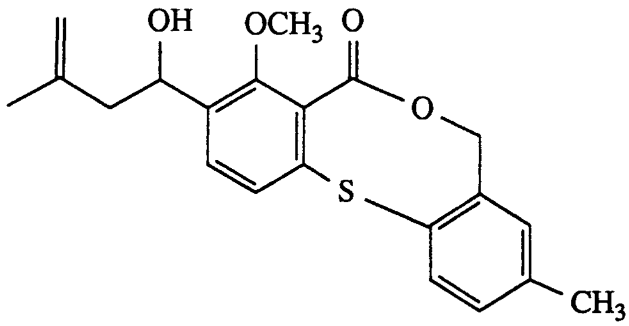

Analog Beispiel II erhält man aus 1,43 g (4,55 mmol) der Verbindung aus Beispiel I und Methallylmagnesiumchlorid 594,9 mg der Titelverbindung.

Ausbeute: 35,3 % der Theorie

$^1$H-NMR ($CDCl_3$) $\delta$ = 1,8 (s, 3H); 2,3 (s, 3H); 2,2-2,5 (m, 2H); 3,95 (S, 3H); 4,85 (m, 1H); 4,95 (m, 1H); 5,15 (m, 3H); 6,85 (s, 1H); 7,1 (d, 1H); 7,35 (d, 1H); 7,45 (D, 1H); 7,65 (d, 1H) ppm.

**Patentansprüche**

**1.** Dibenz-oxa-thiocinone der allgemeinen Formel I

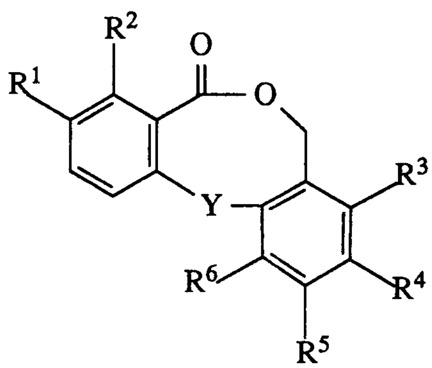

(I)

in welcher

$R^1$ und $R^6$ gleich oder verschieden sind und jeweils
für Wasserstoff stehen, oder
für geradkettiges oder verzweigtes Alkyl oder Alkenyl mit jeweils bis zu 10 Kohlenstoffatomen stehen, die gegebenenfalls 1- bis 3-fach durch Halogen, Azido, Imino oder durch Cycloalkyl mit 3 bis 8 Kohlenstoffatomen oder Aryl mit 6 bis 10 Kohlenstoffatomen substituiert sind, die ihrerseits gegebenenfalls bis zu 2-fach gleich oder verschieden durch Halogen, Nitro, Cyano, Hydroxy oder durch geradkettiges oder verzweigtes Alkyl, Alkoxy oder Alkoxycarbonyl mit jeweils bis zu 6 Kohlenstoffatomen substituiert sind,
oder Alkyl oder Alkenyl, die gegebenenfalls zusätzlich durch eine Gruppe der Formel $-OR^7$, $-CO-R^8$ oder $-CONR^9R^{10}$ substituiert sind,

worin

$R^7$ Wasserstoff, Cycloalkyl mit 3 bis 7 Kohlenstoffatomen oder geradkettiges oder verzweigtes Alkyl, Alkenyl oder Acyl mit jeweils bis zu 8 Kohlenstoffatomen bedeutet, die gegebenenfalls 1- bis 3-fach durch Halogen substituiertes Phenyl, Cycloalkyl mit 3 bis 7 Kohlenstoffatomen, Hydroxy, Halogen oder geradkettiges oder verzweigtes Alkoxy mit bis zu 6 Kohlenstoffatomen oder durch Carboxy, Acyl mit bis zu 6 C-Atomen oder Alkoxycarbonyl mit bis zu 6 C-Atomen substituiert sind,

$R^8$ Wasserstoff, Hydroxy, Phenoxy oder geradkettiges oder verzweigtes Alkoxy mit bis zu 8 Kohlenstoffatomen bedeutet, oder Aryl mit 6 bis 10 Kohlenstoffatomen oder Cycloalkyl mit 3 bis 8 Kohlenstoffatomen bedeutet, die gegebenenfalls durch Hydroxy, Halogen oder durch geradkettiges oder verzweigtes Alkyl, Alkoxy oder Alkoxycarbonyl mit jeweils bis zu 8 Kohlenstoffatomen substituiert sind, geradkettiges oder verzweigtes Alkyl oder Alkenyl mit jeweils bis zu 8 Kohlenstoffatomen bedeutet, die gegebenenfalls durch Halogen Carboxy, Hydroxy oder Alkoxy, Alkoxycarbonyl oder Acyl mit jeweils bis zu 6 C-Atomen substituiert sind,

$R^9$ und $R^{10}$ gleich oder verschieden sind und Wasserstoff, geradkettiges oder verzweigtes Alkyl mit bis zu 8 Kohlenstoffatomen oder Phenyl bedeuten,

oder

$R^1$ und/oder $R^6$ direkt für eine Gruppe der Formel $-OR^7$ oder $-CO-R^8$ stehen,

worin

$R^7$ und $R^8$ die oben angegebene Bedeutung haben,

$R^2$ für Wasserstoff steht, oder für die Gruppe $-OR^7$ steht,

worin

$R^7$ die oben angegebene Bedeutung hat, oder für geradkettiges oder verzweigtes Alkyl oder Alkenyl mit jeweils bis zu 8 Kohlenstoffatomen steht, die gegebenenfalls durch die Gruppe $-OR^7$ substituiert sind,

worin

$R^7$ die oben angegebene Bedeutung hat, oder

für Phenyl steht, das gegebenenfalls 1- bis 3-fach durch Halogen, Nitro, Hydroxy oder durch geradkettiges oder verzweigtes Alkoxy mit bis zu 6 Kohlenstoffatomen substituiert ist,

$R^3$, $R^4$ und $R^5$ gleich oder verschieden sind und für Wasserstoff, Nitro, Halogen, geradkettiges oder verzweigtes Alkyl mit bis zu 8 Kohlenstoffatomen stehen,

Y für ein Schwefelatom oder für die Gruppe der Formel

$>SO$ oder $>SO_2$ steht,

und deren physiologisch unbedenklichen Salze,

mit der Maßgabe, daß wenn Y für die $>SO$- oder $>SO_2$-Gruppe steht,

mindestens einer der Substituenten $R^1$ - $R^6$ verschieden von Wasserstoff sein muß.

**2.** Verbindungen der allgemeinen Formel I gemäß Anspruch 1, in welcher

$R^1$ und $R^6$ gleich oder verschieden sind und für Wasserstoff oder für geradkettiges oder verzweigtes Alkyl oder Alkenyl mit jeweils bis zu 8 Kohlenstoffatomen stehen, die gegebenenfalls bis zu 2-fach durch Fluor, Chlor, Brom, Jod oder durch Cyclopropyl, Cyclopentyl, Cyclohexyl oder Phenyl substituiert sind, die ihrerseits gegebenenfalls durch Fluor, Chlor, Hydroxy oder durch

geradkettiges oder verzweigtes Alkyl oder Alkoxy mit jeweils bis zu 4 Kohlenstoffatomen substituiert sind, oder
Alkyl oder Alkenyl, die gegebenenfalls zusätzlich durch eine Gruppe der Formel $-OR^7$, $-CO-R^8$ oder $-CONR^9R^{10}$ substituiert sind,

worin

$R^7$ — Wasserstoff, Cyclopentyl, Cyclohexyl oder geradkettiges oder verzweigtes Alkyl oder Acyl mit jeweils bis zu 6 Kohlenstoffatomen bedeutet, die gegebenenfalls durch Chlor substituiertes Phenyl, Cyclopropyl, Cyclopentyl, Cyclohexyl, Hydroxy, Fluor, Chlor, Brom, geradkettiges oder verzweigtes Alkoxy mit bis zu 4 Kohlenstoffatomen oder durch Carboxy, Acyl oder Alkoxycarbonyl mit bis zu 6 C-Atomen substituiert sind,

$R^8$ — Wasserstoff, Hydroxy, Phenoxy oder geradkettiges oder verzweigtes Alkoxy mit bis zu 6 Kohlenstoffatomen bedeutet, oder Phenyl, Cyclopropyl, Cyclopentyl oder Cyclohexyl bedeutet, die gegebenenfalls durch Hydroxy, Fluor, Chlor, Brom oder durch geradkettiges oder verzweigtes Alkyl oder Alkoxy mit jeweils bis zu 6 Kohlenstoffatomen substituiert sind,
geradkettiges oder verzweigtes Alkyl mit bis zu 6 Kohlenstoffatomen bedeutet, das gegebenenfalls durch Fluor, Chlor, Brom, Hydroxy, Carboxy oder durch Alkoxy, Acyl oder Alkoxycarbonyl mit bis zu 6 C-Atomen substituiert ist,

$R^9$ und $R^{10}$ — gleich oder verschieden sind und Wasserstoff, geradkettiges oder verzweigtes Alkyl mit bis zu 6 Kohlenstoffatomen bedeuten,

oder

$R^1$ und/oder $R^6$ — direkt für eine Gruppe der Formel $-OR^7$ oder $-CO-R^8$ stehen,

worin

$R^7$ und $R^8$ — die oben angegebene Bedeutung haben,

$R^2$ — für Wasserstoff oder
für die Gruppe $-OR^7$ steht,

worin

$R^7$ — die oben angegebene Bedeutung hat, oder

für geradkettiges oder verzweigtes Alkyl mit bis zu 6 Kohlenstoffatomen steht, das gegebenenfalls durch die Gruppe $-OR^7$ substituiert ist,

worin

$R^7$ — die oben angegebene Bedeutung hat, oder

für Phenyl steht, das gegebenenfalls durch Fluor, Chlor, Brom, Jod, Hydroxy oder geradkettiges oder verzweigtes Alkoxy mit bis zu 4 Kohlenstoffatomen substituiert ist,

$R^3$, $R^4$ und $R^5$ — gleich oder verschieden sind und
für Wasserstoff, Nitro, Fluor, Chlor, Brom, Jod, geradkettiges oder verzweigtes Alkyl mit bis zu 6 Kohlenstoffatomen stehen,

Y — für ein Schwefelatom oder für eine Gruppe der Formel

$>SO$ oder $>SO_2$ steht,

und deren physiologisch unbedenklichen Salze, mit der Maßgabe, daß wenn Y für $>SO$- oder $>SO_2$-Gruppe steht,

mindestens einer der Substituenten $R^1$ - $R^6$ verschieden von Wasserstoff sein muß.

**3.** Verbindungen der allgemeinen Formel I gemäß Anspruch 1, in welcher

$R^1$ und $R^6$ — gleich oder verschieden sind und
für Wasserstoff, oder

für geradkettiges oder verzweigtes Alkyl oder Alkenyl mit bis zu 8 Kohlenstoffatomen stehen, das gegebenenfalls bis zu 2-fach jeweils durch Fluor, Chlor, Brom, Jod oder Phenyl substituiert ist, das seinerseits durch Chlor, Hydroxy, Methyl oder Methoxy substituiert sein kann,
Alkenyl oder Alkyl, die gegebenenfalls zusätzlich durch eine Gruppe der Formel $-OR^7$, $-CO-R^8$ oder $-CONR^9R^{10}$ substituiert sind,

worin

$R^7$ Wasserstoff, Cyclopentyl oder geradkettiges oder verzweigtes Alkyl oder Acyl mit bis zu 4 Kohlenstoffatomen bedeutet, die gegebenenfalls durch Chlor substituiertes Phenyl, Hydroxy, Chlor, Methoxy oder durch Carboxy, Acyl oder Alkoxycarbonyl mit jeweils bis zu 4 C-Atomen substituiert sind,

$R^8$ Wasserstoff, Hydroxy oder geradkettiges oder verzweigtes Alkoxy mit bis zu 4 Kohlenstoffatomen bedeutet, oder
Phenyl, Cyclopropyl, Cyclopentyl oder Cyclohexyl bedeutet, die gegebenenfalls durch Hydroxy, Fluor, Chlor, Brom oder durch geradkettiges oder verzweigtes Alkyl oder Alkoxy mit jeweils bis zu 4 Kohlenstoffatomen substituiert sind, oder
geradkettiges oder verzweigtes Alkyl mit bis zu 4 Kohlenstoffatomen bedeutet, das gegebenenfalls durch Fluor, Chlor, Brom oder durch Hydroxy, Carboxy, Alkoxy, Acyl oder Alkoxycarbonyl mit jeweils bis zu 4 C-Atomen substituiert ist,

$R^9$ und $R^{10}$ gleich oder verschieden sind und Wasserstoff oder geradkettiges oder verzweigtes Alkyl mit bis zu 4 Kohlenstoffatomen bedeuten,

oder

$R^1$ und/oder $R^6$ direkt für eine Gruppe der Formel $-OR^7$ oder $-CO-R^8$ stehen,

worin

$R^7$ und $R^8$ die oben angegebene Bedeutung haben,

$R^2$ für Wasserstoff oder
für die Gruppe $-OR^7$ steht,

worin

$R^7$ die oben angegebene Bedeutung hat, oder

für geradkettiges oder verzweigtes Alkyl mit bis zu 4 Kohlenstoffatomen steht, das gegebenenfalls durch die Gruppe der Formel $-OR^7$ substituiert ist,

worin

$R^7$ die oben angegebene Bedeutung hat, oder

für Phenyl steht, das gegebenenfalls durch Fluor, Chlor, Brom, Jod, Hydroxy oder Methoxy substituiert ist,

$R^3$, $R^4$ und $R^5$ gleich oder verschieden sind und
für Wasserstoff, Nitro, Fluor, Chlor, Brom oder Jod stehen, oder für geradkettiges oder verzweigtes Alkyl mit bis zu 4 Kohlenstoffatomen stehen,

Y für ein Schwefelatom oder für eine Gruppe der Formel

$>SO$ oder $>SO_2$ steht

und deren physiologisch unbedenklichen Salze,

mit der Maßgabe, daß wenn Y für die

$>SO$- oder $>SO_2$-Gruppe steht,

mindestens einer der Substituenten $R^1$ - $R^6$ verschieden von Wasserstoff sein muß.

**4.** Verfahren zur Herstellung von Verbindungen der allgemeinen Formel I

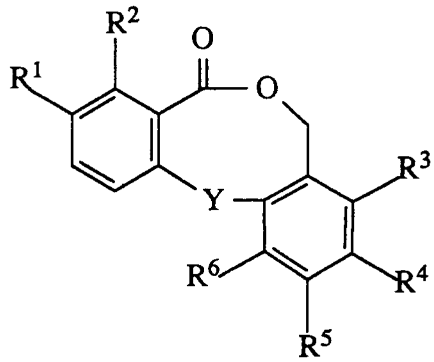

(I)

in welcher

| | |
|---|---|
| $R^1$ und $R^6$ | gleich oder verschieden sind und jeweils für Wasserstoff stehen, oder für geradkettiges oder verzweigtes Alkyl oder Alkenyl mit jeweils bis zu 10 Kohlenstoffatomen stehen, die gegebenenfalls 1- bis 3-fach durch Halogen, Azido, Imino oder durch Cycloalkyl mit 3 bis 8 Kohlenstoffatomen oder Aryl mit 6 bis 10 Kohlenstoffatomen substituiert sind, die ihrerseits gegebenenfalls bis zu 2-fach gleich oder verschieden durch Halogen, Nitro, Cyano, Hydroxy oder durch geradkettiges oder verzweigtes Alkyl, Alkoxy oder Alkoxycarbonyl mit jeweils bis zu 6 Kohlenstoffatomen substituiert sind, oder Alkyl oder Alkenyl, die gegebenenfalls zusätzlich durch eine Gruppe der Formel $-OR^7$, $-CO-R^8$ oder $-CONR^9R^{10}$ substituiert sind, |
| | worin |
| $R^7$ | Wasserstoff, Cycloalkyl mit 3 bis 7 Kohlenstoffatomen oder geradkettiges oder verzweigtes Alkyl, Alkenyl oder Acyl mit jeweils bis zu 8 Kohlenstoffatomen bedeutet, die gegebenenfalls 1- bis 3-fach durch Halogen substituiertes Phenyl, Cycloalkyl mit 3 bis 7 Kohlenstoffatomen, Hydroxy, Halogen oder geradkettiges oder verzweigtes Alkoxy mit bis zu 6 Kohlenstoffatomen oder durch Carboxy, Acyl mit bis zu 6 C-Atomen oder Alkoxycarbonyl mit bis zu 6 C-Atomen substituiert sind, |
| $R^8$ | Wasserstoff, Hydroxy, Phenoxy oder geradkettiges oder verzweigtes Alkoxy mit bis zu 8 Kohlenstoffatomen bedeutet, oder Aryl mit 6 bis 10 Kohlenstoffatomen oder Cycloalkyl mit 3 bis 8 Kohlenstoffatomen bedeutet, die gegebenenfalls durch Hydroxy, Halogen oder durch geradkettiges oder verzweigtes Alkyl, Alkoxy oder Alkoxycarbonyl mit jeweils bis zu 8 Kohlenstoffatomen substituiert sind, geradkettiges oder verzweigtes Alkyl oder Alkenyl mit jeweils bis zu 8 Kohlenstoffatomen bedeutet, die gegebenenfalls durch Halogen Carboxy, Hydroxy oder Alkoxy, Alkoxycarbonyl oder Acyl mit jeweils bis zu 6 C-Atomen substituiert sind, |
| $R^9$ und $R^{10}$ | gleich oder verschieden sind und Wasserstoff, geradkettiges oder verzweigtes Alkyl mit bis zu 8 Kohlenstoffatomen oder Phenyl bedeuten, |
| | oder |
| $R^1$ und/oder $R^6$ | direkt für eine Gruppe der Formel $-OR^7$ oder $-CO-R^8$ stehen, |
| | worin |
| $R^7$ und $R^8$ | die oben angegebene Bedeutung haben, |
| $R^2$ | für Wasserstoff steht, oder für die Gruppe $-OR^7$ steht, |

worin

$R^7$ die oben angegebene Bedeutung hat, oder

für geradkettiges oder verzweigtes Alkyl oder Alkenyl mit jeweils bis zu 8 Kohlenstoffatomen steht, die gegebenenfalls durch die Gruppe $-OR^7$ substituiert sind,

worin

$R^7$ die oben angegebene Bedeutung hat, oder

für Phenyl steht, das gegebenenfalls 1- bis 3-fach durch Halogen, Nitro, Hydroxy oder durch geradkettiges oder verzweigtes Alkoxy mit bis zu 6 Kohlenstoffatomen substituiert ist,

$R^3$, $R^4$ und $R^5$ gleich oder verschieden sind und für Wasserstoff, Nitro, Halogen, geradkettiges oder verzweigtes Alkyl mit bis zu 8 Kohlenstoffatomen stehen,

Y für ein Schwefelatom oder für die Gruppe der Formel

$>SO$ oder $>SO_2$ steht,

und deren physiologisch unbedenklichen Salze,

mit der Maßgabe, daß wenn Y für die $>SO$- oder $>SO_2$-Gruppe steht,

mindestens einer der Substituenten $R^1$ - $R^6$ verschieden von Wasserstoff sein muß, dadurch gekennzeichnet daß man Verbindungen der allgemeinen Formel II

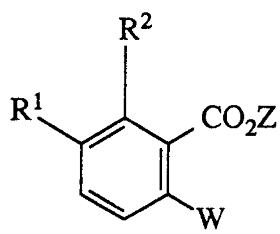

(II)

in welcher

$R^1$ und $R^2$ die oben angegebene Bedeutung haben,

W für Fluor, Chlor, Brom oder Jod, vorzugsweise für Brom steht, und

Z für Wasserstoff, $(C_1$-$C_6)$-Alkyl, Phenyl oder für ein Kalium- oder Natriumkation steht,

mit Verbindungen der allgemeinen Formel (III)

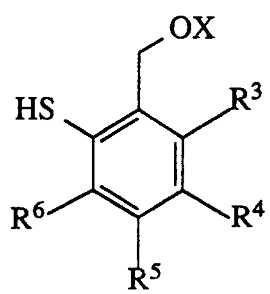

(III)

in welcher

| | |
|---|---|
| $R^3$, $R^4$, $R^5$ und $R^6$ | die oben angegebene Bedeutung haben, und |
| X | für eine typische Hydroxyschutzgruppe, beispielsweise Tetrahydropyranyl steht, |

zunächst unter Abspaltung von Halogenwasserstoffsäuren, vorzugsweise von Bromwasserstoffsäure, zu Verbindungen der allgemeinen Formel (IV)

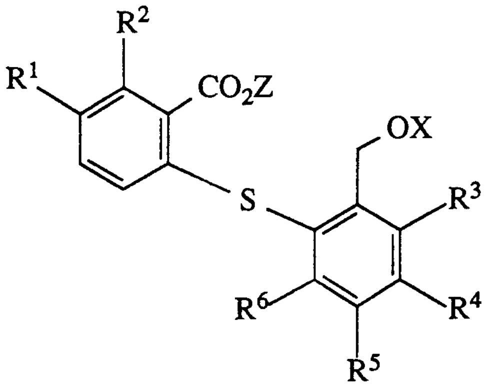

(IV)

in welcher

$R^1$, $R^2$, $R^3$, $R^4$, $R^5$, $R^6$, X und Z die oben angegebene Bedeutung haben,

in inerten Lösemitteln kondensiert, anschließend die Hydroxylgruppe nach üblicher Methode deblockiert und unter Wasserabspaltung cyclisiert, wobei sowohl die Kondensation als auch die Cyclisierung gegebenenfalls in Anwesenheit einer Base, eines Hilfsstoffes und/oder eines Katalysators durchgeführt werden kann und wobei die Substituenten $R^1$ - $R^6$ sowohl vor der Kondensation in die Verbindungen der allgemeinen Formeln (II) und (III), als auch nach der Cyclisierung in die Verbindungen der allgemeinen Formel (IV) nach üblichen Methoden, wie beispielsweise Substitution, Addition oder Eliminierung eingeführt und gegebenenfalls anschließend in andere funktionelle Gruppen überführt werden, und im Fall, daß Y für die

$>$SO- oder $>SO_2$-Gruppe steht, die Dibenz-oxa-thiocinone (Y = S)

nach üblicher Methode, beispielsweise mit Persäuren, wie m-Chlorperbenzoesäure, oder mit anderen üblichen Oxidationsmitteln wie beispielsweise Wasserstoffperoxid oder Natriummetaperjodat oxidiert werden.

**5.** Verbindungen der allgemeinen Formel I

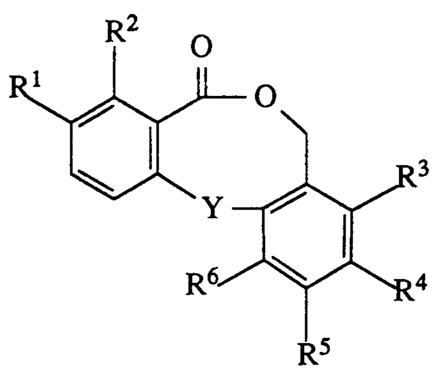

(I)

in welcher

| | |
|---|---|
| $R^1$ und $R^6$ | gleich oder verschieden sind und jeweils<br>für Wasserstoff stehen, oder<br>für geradkettiges oder verzweigtes Alkyl oder Alkenyl mit jeweils bis zu 10 Kohlenstoffatomen stehen, die gegebenenfalls 1- bis 3-fach durch Halogen, Azido, Imino oder durch Cycloalkyl mit 3 bis 8 Kohlenstoffatomen oder Aryl mit 6 bis 10 Kohlenstoffatomen substituiert sind, die ihrerseits gegebenenfalls bis zu 2-fach gleich oder verschieden durch Halogen, Nitro, Cyano, Hydroxy oder durch geradkettiges oder verzweigtes Alkyl, Alkoxy oder Alkoxycarbonyl mit jeweils bis zu 6 Kohlenstoffatomen substituiert sind,<br>oder Alkyl oder Alkenyl, die gegebenenfalls zusätzlich durch eine Gruppe der Formel $-OR^7$, $-CO-R^8$ oder $-CONR^9R^{10}$ substituiert sind, |
| | worin |
| $R^7$ | Wasserstoff, Cycloalkyl mit 3 bis 7 Kohlenstoffatomen oder geradkettiges oder verzweigtes Alkyl, Alkenyl oder Acyl mit jeweils bis zu 8 Kohlenstoffatomen bedeutet, die gegebenenfalls 1- bis 3-fach durch Halogen substituiertes Phenyl, Cycloalkyl mit 3 bis 7 Kohlenstoffatomen, Hydroxy, Halogen oder geradkettiges oder verzweigtes Alkoxy mit bis zu 6 Kohlenstoffatomen oder durch Carboxy, Acyl mit bis zu 6 C-Atomen oder Alkoxycarbonyl mit bis zu 6 C-Atomen substituiert sind, |
| $R^8$ | Wasserstoff, Hydroxy, Phenoxy oder geradkettiges oder verzweigtes Alkoxy mit bis zu 8 Kohlenstoffatomen bedeutet, oder<br>Aryl mit 6 bis 10 Kohlenstoffatomen oder Cycloalkyl mit 3 bis 8 Kohlenstoffatomen bedeutet, die gegebenenfalls durch Hydroxy, Halogen oder durch geradkettiges oder verzweigtes Alkyl, Alkoxy oder Alkoxycarbonyl mit jeweils bis zu 8 Kohlenstoffatomen substituiert sind, geradkettiges oder verzweigtes Alkyl oder Alkenyl mit jeweils bis zu 8 Kohlenstoffatomen bedeutet, die gegebenenfalls durch Halogen Carboxy, Hydroxy oder Alkoxy, Alkoxycarbonyl oder Acyl mit jeweils bis zu 6 C-Atomen substituiert sind, |
| $R^9$ und $R^{10}$ | gleich oder verschieden sind und<br>Wasserstoff, geradkettiges oder verzweigtes Alkyl mit bis zu 8 Kohlenstoffatomen oder Phenyl bedeuten, |
| | oder |
| $R^1$ und/oder $R^6$ | direkt für eine Gruppe der Formel $-OR^7$ oder $-CO-R^8$ stehen, |
| | worin |
| $R^7$ und $R^8$ | die oben angegebene Bedeutung haben, |
| $R^2$ | für Wasserstoff steht, oder für die Gruppe $-OR^7$ steht, |
| | worin |
| $R^7$ | die oben angegebene Bedeutung hat, oder |
| | für geradkettiges oder verzweigtes Alkyl oder Alkenyl mit jeweils bis zu 8 Kohlenstoffatomen steht, die gegebenenfalls durch die Gruppe $-OR^7$ substituiert sind, |
| | worin |
| $R^7$ | die oben angegebene Bedeutung hat, oder |
| | für Phenyl steht, das gegebenenfalls 1- bis 3-fach durch Halogen, Nitro, Hydroxy oder durch geradkettiges oder verzweigtes Alkoxy mit bis zu 6 Kohlenstoffatomen substituiert ist, |
| $R^3$, $R^4$ und $R^5$ | gleich oder verschieden sind und<br>für Wasserstoff, Nitro, Halogen, geradkettiges oder verzweigtes Alkyl mit bis zu 8 Kohlenstoffatomen stehen, |
| Y | für ein Schwefelatom oder für die Gruppe der Formel |

$>SO$ oder $>SO_2$ steht,

und deren physiologisch unbedenklichen Salze,

zur Verwendung bei der Bekämpfung von Erkrankungen.

**6.** Arzneimittel enthaltend mindestens eine Verbindung der allgemeinen Formel I gemäß Anspruch 1.

**7.** Verfahren zur Herstellung von Arzneimitteln, dadurch gekennzeichnet, daß man Verbindungen der allgemeinen Formel I gemäß Anspruch 1 gegebenenfalls unter Verwendung von üblichen Hilfs- und Trägerstoffen in eine geeignete Applikationsform überführt.

**8.** Verwendung von Verbindungen der allgemeinen Formel I gemäß Anspruch 1 bei der Herstellung von kreislaufwirksamen Arzneimitteln.

Europäisches Patentamt

# EUROPÄISCHER RECHERCHENBERICHT

Nummer der Anmeldung

EP 91 12 0707

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | JOURNAL OF THE AMERICAN CHEMICAL SOCIETY, Bd. 103, Nr. 22, 14. Januar 1981, Washington, DC, US, Seiten 120 - 127; W.Y. LAM ET AL.: 'Diaryltetraoxypersulphuranes: preparation, characterisation, and reactions' * Verbindungen 10,36,37 * * Seite 121, linke Spalte, Absatz 6 - rechte Spalte, Absatz 2 * | 1 | C07D327/02 |
| A | TETRAHEDRON LETTERS, Nr. 45, November 1974, Oxford, GB, Seiten 3941 - 3942; T. SASSA ET AL.: 'Structure of penicillide, a new metabolite produced by a Penicillium sp.' * das ganze Dokument * | 1 | |
| P,A | DE-A-3 919 255 (BAYER) * Seite 2 - Seite 3 * | 1,5-8 | |
| | | | RECHERCHIERTE SACHGEBIETE (Int. Cl.5) |
| | | | C07D |

Der vorliegende Recherchenbericht wurde für alle Patentansprüche erstellt

| Recherchenort | Abschlußdatum der Recherche | Prüfer |
|---|---|---|
| DEN HAAG | 17 FEBRUAR 1992 | ENGLISH R.F. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)